# FASCICULE DE BREVET EUROPEEN

(11) **EP 4 337 222 B1**
(45) Date de publication et mention de la délivrance du brevet: **28.10.2026**
(21) Numéro de dépôt: 22728908.9
(22) Date de dépôt: 13.05.2022
(51) Int. Cl.: A61K 31/7072, A61K 9/00, A61K 9/107, A61P 17/02, A61P 25/28, C07H 19/06

(54) **PRODROGUE NUCLÉOLIPIDIQUE ANTIOXYDANTE, COMPOSITION PHARMACEUTIQUE POUR SON ADMINISTRATION ET LEURS UTILISATIONS THÉRAPEUTIQUES**
ANTIOXIDATIVE NUKLEOLIPID-PRODRUGS, PHARMAZEUTISCHE ZUSAMMENSETZUNG ZUR VERABREICHUNG DAVON UND THERAPEUTISCHE VERWENDUNGEN DAVON
ANTIOXIDANT NUCLEOLIPID PRODRUG, PHARMACEUTICAL COMPOSITION FOR ADMINISTRATION THEREOF, AND THERAPEUTIC USES THEREOF

(30) Priorité: 14.05.2021 FR 2105088
(43) Date de publication de la demande: 20.03.2024
(73) Titulaire: UNIVERSITE DE BORDEAUX, 33000 Bordeaux (FR); Centre National de la Recherche Scientifique, 75016 Paris (FR); INSERM (Institut National de la Santé et de la Recherche Médicale), 75013 Paris (FR); Centre Hospitalier Universitaire de Bordeaux, 33404 Talence (FR)
(72) Inventeur: DESVERGNES, Valérie, 33140 Villenave d'Ornon (FR); CRAUSTE-MANCIET, Sylvie, 33000 Bordeaux (FR); KLUFTS-EDEL, Arthur, 68180 Horbourg-Wihr (FR)
(74) Mandataire: Santarelli
(86) Numéro de dépôt international: PCT/EP2022/063064
(87) Numéro de publication internationale: WO 2022/238568

(56) Documents cités:
- WO-A1-2016/057825
- WO-A1-2020/247912
- SINOKROT HANADI ET AL: "Advanced Prodrug Strategies in Nucleoside and Non-Nucleoside Antiviral Agents: A Review of the Recent Five Years", MOLECULES, vol. 22, no. 10, 1 October 2017 (2017-10-01), DE, pages 1736, XP055869773, ISSN: 1433-1373, DOI: 10.3390/molecules22101736
- ALADIN HAMOUD ET AL: "An organocatalyzed Stetter reaction as a bio-inspired tool for the synthesis of nucleic acid-based bioconjugates", ORGANIC & BIOMOLECULAR CHEMISTRY, vol. 16, no. 10, 1 January 2018 (2018-01-01), pages 1760 - 1769, XP055754024, ISSN: 1477-0520, DOI: 10.1039/C8OB00192H
- LASKIN JEFFREY D. ET AL: "Oxidants and antioxidants in sulfur mustard-induced injury : Sulfur mustard and oxidative stress", ANNALS OF THE NEW YORK ACADEMY OF SCIENCES, vol. 1203, no. 1, 1 August 2010 (2010-08-01), US, pages 92 - 100, XP055870652, ISSN: 0077-8923, DOI: 10.1111/j.1749-6632.2010.05605.x
- YE FENG ET AL: "Antioxidant Therapies for Alzheimer's Disease", OXIDATIVE MEDICINE AND CELLULAR LONGEVITY, vol. 2012, 1 January 2012 (2012-01-01), US, pages 1 - 17, XP055380303, ISSN: 1942-0900, DOI: 10.1155/2012/472932
- DE LEEUW FRANCISCA A. ET AL: "Brain [gamma]-Tocopherol Levels Are Associated with Presynaptic Protein Levels in Elderly Human Midfrontal Cortex", vol. 77, no. 2, 15 September 2020 (2020-09-15), NL, pages 619 - 627, XP055870656, ISSN: 1387-2877, Retrieved from the Internet <URL:https://www.ncbi.nlm.nih.gov/pmc/articles/PMC7592653/pdf/jad-77-jad200166.pdf> DOI: 10.3233/JAD-200166

## Description

La présente invention s'inscrit dans le domaine des applications thérapeutiques des molécules pharmaceutiquement actives à activité antioxydante.

Plus particulièrement, la présente invention concerne une prodrogue à base d'une molécule antioxydante pharmaceutiquement active, ainsi qu'une composition pharmaceutique contenant une telle prodrogue. L'invention concerne également des applications thérapeutiques de cette prodrogue et de cette composition pharmaceutique.

Les composés antioxydants sont largement utilisés dans le domaine médical pour la prévention et le traitement de nombreuses pathologies, tirant profit de leur capacité à inhiber l'oxydation d'autres molécules et notamment à les protéger de l'action des espèces réactives de l'oxygène, en particulier des radicaux libres, et du stress oxydatif, impliqués dans le développement de ces pathologies.

L'effet bénéfique des composés antioxydants a notamment été démontré dans le cadre de la réparation des brûlures cutanées ou pour le traitement préventif ou curatif de maladies ophtalmiques, telles que la dégénérescence maculaire liée à l'âge (DMLA) ou la cataracte. La publication de Laskin et al., Annals of the New York Academy of Sciences, 2010, 92-100, décrit l'effet de protection des molécules ciblant les voies du stress oxydatif contre les brûlures induites par le gaz moutarde.

Il a également été décrit dans l'art antérieur, par exemple dans la publication de Delanty et Dichter, Arch. Neurol. 2000, 57, 1265-1270, que les composés antioxydants permettent de prévenir, retarder ou améliorer de nombreux désordres neurologiques. La publication de de Leeeuw et al., Alzheimer's Dement. 2020, DOI : 10.1002/trc2.12021, la publication de de Leeuw et al., dans Journal of Alzheimer's disease, 2020, 619-627, la publication de Ye Feng et al., Oxidative Medecine and Cellular Longevity, 2012, 1-17, ou encore la publication de Moneim, Current Alzheimer Research, 2015, 12, 335-349, indiquent plus précisément que les antioxydants, et pour certaines notamment la vitamine E, et en particulier l'α-tocophérol, jouent un rôle dans la prévention et le traitement de la maladie d'Alzheimer.

La délivrance des molécules pharmaceutiquement actives en général, et des molécules antioxydantes en particulier, à leurs cibles pharmacologiques est un des principaux enjeux de la chimie médicinale et de la galénique. De nombreux systèmes ont ainsi été proposés par l'art antérieur pour transporter les principes actifs dans l'organisme, et favoriser leur passage au travers des membranes biologiques, tout en les protégeant contre une possible dégradation avant d'avoir atteint leur cible. On peut citer à cet égard, à titre d'exemples, les nanovecteurs, les dendrimères, les capsules thérapeutiques, etc.

La présente invention vise à proposer un système permettant de véhiculer efficacement les molécules antioxydantes pharmaceutiquement actives au sein de l'organisme, et notamment de promouvoir leur passage au travers des membranes biologiques, de préférence y compris au travers de la membrane hémato-encéphalique, jusqu'à l'intérieur des neurones, et ce que les molécules antioxydantes présentent un caractère lipophile ou hydrophile, et tout en assurant qu'elles restent stables jusqu'à atteindre leur cible.

A cet effet, les présents inventeurs se sont intéressés au principe des prodrogues, selon lequel une molécule pharmaceutiquement active est conjuguée à un composé jouant le rôle de vecteur pour son transport dans l'organisme, vecteur dont elle est ensuite séparée, par un processus biologique, pour être libérée dans l'organisme sous sa forme pharmaceutiquement active.

Le document WO 2016/057825 décrit des prodrogues pour la délivrance intracellulaire d'un nucléoside monophosphorylé, dans lesquelles un nucléoside ou un analogue de nucléoside tel que la 5-azacytidine est conjugué à la vitamine E par le biais d'une liaison phosphodiester ou phosphoramidate.

La publication de Sinokrot Hanadi et al., Molecules, 2017, 1736 présente différentes stratégies pour améliorer la délivrance des agents antiviraux de type nucléosidique.

Dans un tout autre domaine, celui de la délivrance des molécules antioxydantes, les présents inventeurs ont découvert que la mise en œuvre d'un vecteur de structure chimique spécifique, du type dérivé d'un nucléoside rendu lipidique par fixation d'un groupement lipidique, s'avère être un particulièrement bon promoteur d'absorption de sorte qu'il permet le passage des membranes biologiques par les molécules antioxydantes de nature aussi bien lipophile qu'hydrophile. Ce vecteur particulier permet en outre avantageusement de stabiliser les molécules antioxydantes, en évitant leur dégradation, notamment leur oxydation, dans l'organisme, tout en leur maintenant / conférant une bonne biodisponibilité permettant notamment leur administration par voie orale.

Ainsi, selon un premier aspect, la présente invention concerne une prodrogue consistant en :
- un composé, dit composé actif, comprenant :
   une molécule antioxydante pharmaceutiquement active couplée de manière covalente, par une liaison comprenant au moins un groupe ester carboxylique, à un nucléoside comprenant un motif ribose ou désoxyribose lié de manière covalente à une base nucléique,
   ledit nucléoside portant au moins un groupement, dit groupement lipidant, choisi parmi les groupements -R et -CO-R, dans lesquels R représente un radical hydrocarboné, de préférence en C1-C55, préférentiellement en C1-C25 et préférentiellement encore en C10-C25, linéaire, ramifié et/ou cyclique, saturé et/ou insaturé, aromatique ou non, éventuellement substitué, éventuellement interrompu par un ou plusieurs hétéroatomes radical hydrocarboné, tels que les atomes d'oxygène, d'azote et de soufre, et/ou par un ou plusieurs groupements comprenant au moins un hétéroatome ou au moins un groupement comprenant au moins un hétéroatome, R ne comprenant cependant aucun groupement hydroxyle, amine primaire, amine secondaire, phosphate ou thiol, ledit radical hydrocarboné pouvant comporter un cycle ou plusieurs cycles, le cas échéant un ou plusieurs hétérocycles comprenant un ou plusieurs hétéroatomes tels que les atomes d'oxygène, d'azote ou de soufre, lesdits cycles étant optionnellement condensés,
   le groupement lipidant pouvant optionnellement être lié par liaison covalente simultanément à deux atomes distincts du nucléoside,
- ou un des sels pharmaceutiquement acceptables de ce composé actif.

Dans le composé actif selon l'invention, en outre :
- chacun des groupements hydroxyles dudit motif ribose ou désoxyribose non impliqué dans une liaison à la molécule antioxydante ou à un groupement lipidant est substitué par un groupement protecteur d'une fonction hydroxyle fixé sur l'atome d'oxygène dudit groupement hydroxyle et choisi parmi les radicaux hydrocarbonés linéaires, ramifiés et/ou cycliques, saturés et/ou insaturés, aromatiques ou non, éventuellement substitués, éventuellement interrompus par un ou plusieurs hétéroatomes, tels que les atomes d'oxygène, d'azote et de soufre, et/ou par un ou plusieurs groupements comprenant au moins un hétéroatome ou au moins un groupement comprenant au moins un hétéroatome, le groupement protecteur d'une fonction hydroxyle ne comportant aucun groupement hydroxyle, amine primaire, amine secondaire, phosphate ou thiol, les radicaux hydrocarbonés pouvant comporter un cycle ou plusieurs cycles, le cas échéant un ou plusieurs hétérocycles comprenant un ou plusieurs hétéroatomes, tels que les atomes d'oxygène, d'azote ou de soufre, lesdits cycles étant optionnellement condensés. Des groupements protecteurs d'une fonction hydroxyle utilisables selon l'invention sont les éthers ou esters d'alkyle, d'allyle, de propargyle, de cycloalkyle, d'aryle (tels que benzyle, naphthyle, etc.) ou hétéroaryle (tels que pyridine, indole, benzothiophène, benzofurane, etc.) ou de polyéthylène glycol, les esters dérivés du cholestérol et les orthoformiates. Des exemples particuliers de tels groupements sont les groupements éther ou ester de tert-butyle, vinyle, benzyle, méthoxyméthyle, tétrahydropyranyle, triisopropylsilyle, tert-butyldiméthylsilyle, tert-butyldiphényl silyle, benzoyle, acétyle, pivalate, glycéryle, etc. Des groupements protecteurs d'une fonction hydroxyle tels que les acétals et acétonides peuvent autrement être utilisés selon l'invention pour protéger simultanément deux fonctions hydroxyles du nucléoside ;
- et chacun des groupements amines primaires non impliqué dans une liaison à ladite molécule antioxydante ou à un groupement lipidant est substitué par un groupement protecteur d'une fonction amine primaire fixé sur l'atome d'azote dudit groupement amine primaire et choisi parmi les radicaux hydrocarbonés linéaires, ramifiés et/ou cycliques, saturés et/ou insaturés, aromatiques ou non, éventuellement substitués, éventuellement interrompus par un ou plusieurs hétéroatomes, tels que les atomes d'oxygène, d'azote et de soufre, et/ou par un ou plusieurs groupements comprenant au moins un hétéroatome ou au moins un groupement comprenant au moins un hétéroatome, le groupement protecteur d'une fonction amine primaire ne comportant aucun groupement hydroxyle, amine primaire, amine secondaire, phosphate ou thiol, les radicaux hydrocarbonés pouvant comporter un cycle ou plusieurs cycles, le cas échéant un ou plusieurs hétérocycles comprenant un ou plusieurs hétéroatomes tels que les atomes d'oxygène, d'azote ou de soufre, lesdits cycles étant optionnellement condensés. Des groupements protecteurs d'une fonction amine primaire utilisables selon l'invention sont les alkyles, les allyles, les propargyles, les cycloalkyles, les aryles (tels que benzyle, naphthyle, etc.), les polyéthylène glycols, les amides d'alkyle ou d'aryle, les acétamides (tels que benzèneacétamide, pyridine acétamide, etc.), les carbamates, les azotures, les triazoles . Des exemples particuliers de tels groupements sont les groupements tert-butyle, vinyle, benzyle, méthoxyméthyle, tétrahydropyranyle, triisopropylsilyle, tert-butyldiméthylsilyle, tert-butyldiphényl silyle, benzoyle, acétyle, pivalate, glycéryle, etc.

Ainsi, le nucléoside du composé actif selon l'invention ne comporte aucun groupement hydroxyle libre ni aucun groupement amine primaire libre.

On entend dans la présente description, par le terme « prodrogue », de manière classique en elle-même, un dérivé pharmaceutiquement acceptable d'une substance pharmaceutiquement active, dont la transformation *in vivo* libère la substance pharmaceutiquement active.

On entend, par molécule « pharmaceutiquement active », une molécule à usage thérapeutique, indiquée pour prévenir et/ou traiter une maladie, et/ou en prévenir et/ou en améliorer un ou plusieurs des symptômes.

On entend, par dérivé « pharmaceutique acceptable », tout dérivé mettant en œuvre, conjuguée avec la molécule pharmaceutiquement active, une substance ne provoquant aucun effet adverse, allergique ou autre réaction indésirable quand elle est administrée à un sujet, en particulier à un mammifère, notamment à un humain, sous une forme conjuguée avec la molécule pharmaceutiquement active.

On entend en outre dans la présente description, par « sel pharmaceutiquement acceptable », tout sel du composé actif mettant en œuvre, en tant que contre-ion, une espèce ne produisant aucun effet adverse, allergique ou autre réaction indésirable quand il est administré à un sujet, en particulier à un mammifère.

Tout sel non toxique classiquement utilisé dans le domaine pharmaceutique peut être mis en œuvre dans le cadre de l'invention, qu'il soit formé à partir d'acides organiques ou à partir d'acides inorganiques, par exemple un chlorure, un bromure, un formiate, un acétate, etc. Ce sel peut être synthétisé à partir du composé actif et de l'acide correspondant, selon toute méthode chimique conventionnelle.

Préférentiellement, le sel du composé actif est choisi pour être soluble dans les phases huileuses.

On désigne dans la présente description, par l'expression « nucléoside lipidisé », un nucléoside modifié par au moins un groupement lipidant conformément à l'invention, un tel groupement conférant au nucléoside un caractère lipidique.

Comme indiqué ci-avant, le conjugué formé par la molécule antioxydante et le nucléoside portant un groupe lipidant conforme à l'invention présente une capacité d'absorption dans les membranes biologiques particulièrement bonne. En outre, la présence d'une liaison comprenant un groupe ester carboxylique, entre la molécule antioxydante active et le nucléoside lipidisé, permet avantageusement une libération *in situ* de la molécule antioxydante sous sa forme active dans tous les sites cibles de l'organisme, par clivage de la liaison ester carboxylique par hydrolyse par les estérases, enzymes ubiquitaires présentes dans toutes les cellules de l'organisme.

Sous sa forme du composé actif, ou d'un sel de ce composé actif soluble dans les phases huileuses, la prodrogue selon l'invention est avantageusement soluble dans les véhicules lipophiles, ce qui facilite son intégration au sein de nombreuses formes galéniques, et en particulier dans les émulsions, et notamment les nanoémulsions, comprenant une phase lipophile et une phase aqueuse, l'une de ces phases étant dispersée dans l'autre phase sous forme de fines gouttelettes.

Le nucléoside entrant dans la constitution du composé actif selon l'invention peut porter un ou plusieurs groupements lipidants, qui lui sont fixés par liaison covalente. Dans le cas d'une pluralité de groupements lipidants, ces derniers peuvent être identiques les uns aux autres, ou différents les uns des autres.

Dans des modes de réalisation particuliers de l'invention, au moins un groupement lipidant, le cas échéant chaque groupement lipidant, est choisi parmi :
- les groupements -CH₂-R', -CO-CH₂-R' et -CO-(CH₂)₂-CO-O-R', où R' représente le cholestérol, le tocophérol, le tocotriénol, un noyau phényle éventuellement substitué par un ou plusieurs groupements R₅, un noyau naphtyle éventuellement substitué par un ou plusieurs groupements R₅, un noyau pyridine éventuellement substitué par un ou plusieurs groupements R₅, où R₅ représente une chaine hydrocarbonée linéaire ou ramifiée, saturée ou insaturée en C1-C25, de préférence en C4-C25 et préférentiellement en C10-C25,
- un radical allyle ou un radical propargyle,
- les groupements -R et -CO-R, où R représente : une chaine hydrocarbonée linéaire ou ramifiée, saturée ou insaturée en C1-C25, de préférence en C4-C25 et préférentiellement en C10-C25 ; un groupement -(CH₂)₂-O-R₅-O-CH₃ où R₅ représente une chaine hydrocarbonée linéaire ou ramifiée, saturée ou insaturée en C1-C25, de préférence en C4-C25 et préférentiellement en C10-C25 ; un groupement -CH₂-CH(O-R₆)-O-R₇ où R₆ et R₇, identiques ou différents, représentent chacun une chaine hydrocarbonée linéaire ou ramifiée, saturée ou insaturée en C1-C25, de préférence en C4-C25 et préférentiellement en C10-C25 ; un groupement -CH(O-R₆)-O-R₇ où R₆ et R₇, identiques ou différents, représentent chacun une chaine hydrocarbonée linéaire ou ramifiée, saturée ou insaturée en C1-C25, de préférence en C4-C25 et préférentiellement en C10-C25 ; ou un groupement -CH₂-CH(O-CO-R₆)-O-CO-R₇ où R₆ et R₇, identiques ou différents, représentent chacun une chaine hydrocarbonée linéaire ou ramifiée, saturée ou insaturée en C1-C25, de préférence en C4-C25 et préférentiellement en C10-C25,
- les groupements -CH₂-R₈ où R₈ représente un groupement triazole, un groupement benzothiophène, un groupement benzofurane ou un groupement indole (benzopyrrole), R₈ étant éventuellement substitué par un ou plusieurs groupements R₅ où R₅ représente une chaine hydrocarbonée linéaire ou ramifiée, saturée ou insaturée en C1-C25, de préférence en C4-C25 et préférentiellement en C10-C25,
- les groupements -(CH₂)₂-O-(CH₂)₂-O-(CH₂)ₚ-O-CH₂-R₁₅, où p est un nombre entier compris entre 2 et 25 et R₁₅ représente un atome d'hydrogène ou un groupement phényle ;
- les groupements -CO-CH₂-O-(CH₂)_{q}-O-CH₂-R₁₆, où q est un nombre entier compris entre 2 et 25 et R₁₆ représente un atome d'hydrogène ou un groupement phényle ;
- un groupement triazole, le cas échéant substitué par une chaine hydrocarbonée linéaire ou ramifiée, saturée ou insaturée en C1-C25, de préférence en C4-C25 et préférentiellement en C10-C25, par exemple par un radical géranyle ou farnésyle.

Autrement, le groupement lipidant peut former un groupement acétal avec deux atomes d'oxygène distincts du motif ribose du nucléoside, ce groupement acétal comprenant une ou plusieurs chaines hydrocarbonée(s) linéaire(s), ramifiée(s) et/ou cycliques, saturée(s) ou insaturée(s), en C1-C25, aromatiques ou non, éventuellement substituées, éventuellement interrompues par un ou plusieurs hétéroatomes et/ou par un ou plusieurs groupements comprenant au moins un hétéroatome ou au moins un groupement comprenant au moins un hétéroatome, à l'exclusion des groupements hydroxyle, amine primaire, amine secondaire, phosphate et thiol, et pouvant comporter un cycle ou plusieurs cycles, le cas échéant un ou plusieurs hétérocycles comprenant un ou plusieurs hétéroatomes, les cycles étant optionnellement condensés.

Ce groupement acétal peut par exemple répondre à l'une des formules générales suivantes :
- -O-CH(-O-)-O-R₅, où R₅ représente une chaine hydrocarbonée linéaire ou ramifiée, saturée ou insaturée en C1-C25, de préférence en C4-C25 et préférentiellement en C10-C25 ;
- O-C(-O-)-R₁₇, où R₁₇ forme avec l'atome de carbone auquel il est lié un cycloalkyle comportant 3 à 25 atomes de carbone ;
- O-C(-O-)(-R₆)-R₇, où R₆ et R₅, identiques ou différents, représentent chacun une chaine hydrocarbonée linéaire ou ramifiée, saturée ou insaturée en C1-C25, de préférence en C4-C25 et préférentiellement en C10-C25.

De tels groupements lipidants, associés à l'absence de groupements hydroxyles libres et de groupements amines primaires libres au sein du nucléoside, assurent une solubilité de la prodrogue selon l'invention dans les véhicules huileux qui est particulièrement bonne.

Des exemples de groupements lipidants utilisables dans le cadre de l'invention répondent aux formules générales respectives (IVa) à (IVq) : 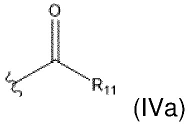 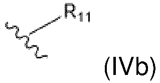 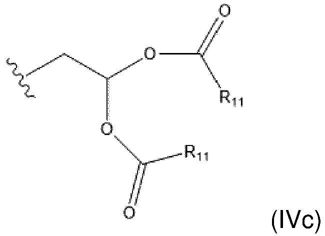 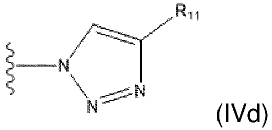 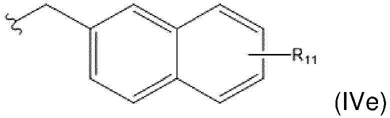 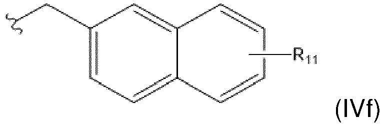 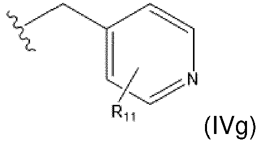 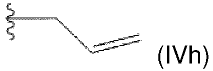 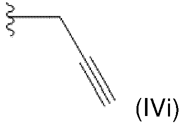 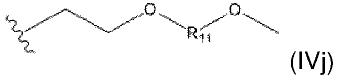 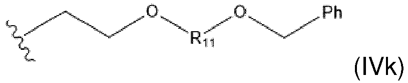 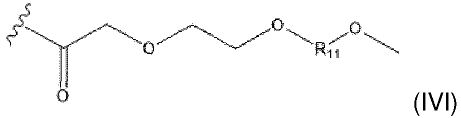 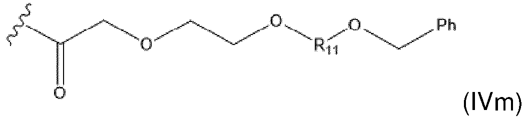 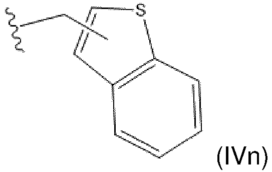 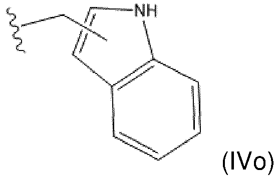 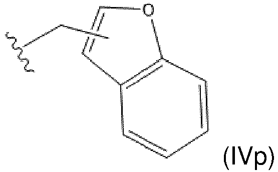 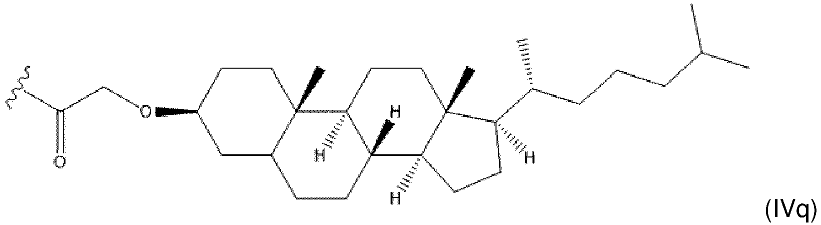 dans lesquelles R₁₁ représente une chaine hydrocarbonée linéaire ou ramifiée, saturée ou insaturée, en C1-C25, de préférence en C4-C25 et préférentiellement en C10-C25.

Un nucléoside est défini dans la présente description de manière classique en elle-même, comme une entité présentant la formule générale (V) : 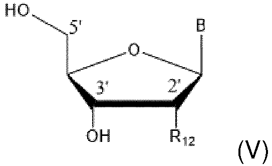 dans laquelle B représente une base nucléique, également nommée nucléobase ou base azotée, et R₁₂ représente un groupement hydroxyle ou un atome d'hydrogène.

Préférentiellement, le nucléoside selon l'invention est un désoxyribonucléoside, dans lequel, dans la formule ci-dessus, R₁₂ représente un atome d'hydrogène.

Selon l'invention, le nucléoside conjugué à la molécule antioxydante est modifié par fixation covalente d'au moins un groupement lipidant conforme à l'invention et par protection de chacun de ses groupements hydroxyles restant libres éventuels par un groupement protecteur d'une fonction hydroxyle, tel que défini ci-avant, ainsi que de chacun de ses groupements amines primaires restant libres éventuels par un groupement protecteur d'une fonction amine primaire, tel que défini ci-avant.

Le nucléoside ainsi modifié par le groupement lipidant, qualifié dans la présente description de nucléoside lipidé, ou nucléolipide, est avantageusement non toxique, en particulier pour les mammifères, notamment pour l'homme, et biodégradable.

La base nucléique entrant dans la constitution du nucléoside selon l'invention peut être :
- une base azotée naturelle, de type purique ou pyrimidique, optionnellement substituée, en particulier par un atome d'halogène, par exemple un atome de brome ou d'iode, ou par un radical hydrocarboné linéaire, ramifié et/ou cyclique, saturé et/ou insaturé, aromatique ou non, éventuellement substitué, éventuellement interrompu par un ou plusieurs hétéroatomes et/ou par un ou plusieurs groupements comprenant au moins un hétéroatome ou au moins groupement comprenant au moins un hétéroatome, à l'exclusion des groupements hydroxyle, amine primaire, amine secondaire, phosphate ou thiol, et pouvant comporter un cycle ou plusieurs cycles, optionnellement condensés, par exemple substituée par un radical éthynyle ou un noyau triazole éventuellement substitué, par exemple par une chaine hydrocarbonée linéaire ou ramifiée, saturée ou insaturée en C1-C25,
- ou une base hétérocyclique non naturelle, monocyclique ou bicyclique, dont chaque cycle comporte de 4 à 7 chainons,
cette base nucléique étant le cas échéant modifiée pour ne pas comporter de groupement amine primaire libre.

La base nucléique du nucléoside selon l'invention peut être choisie parmi l'adénine, la guanine, l'uracile, la thymine et la cytosine, ou leurs dérivés, de formules respectives (Vla) à (Vle) : 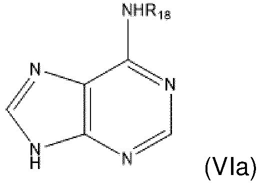 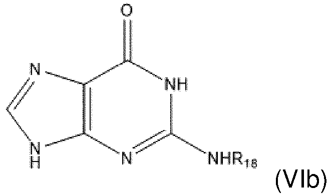 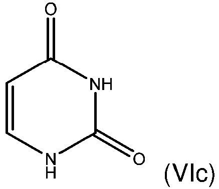 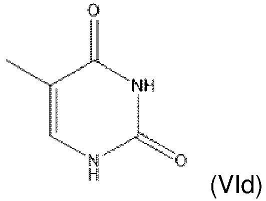 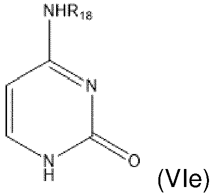 où R₁₈, s'il ne représente pas un groupement lipidant conforme à l'invention, représente un groupement protecteur d'une fonction amine libre tel que défini ci-avant.

Chacune de ces bases nucléiques peut être modifiée, au niveau d'une fonction amine primaire ou secondaire, par un groupement lipidant conforme à l'invention.

La liaison de la base nucléique au motif ribose ou désoxyribose au sein du nucléoside selon l'invention est réalisée selon la configuration naturelle des nucléosides, c'est-à-dire entre l'atome de carbone en position 1 du motif ribose ou désoxyribose et l'atome d'azote en position 1 pour les pyrimidines et en position 9 pour les purines.

Le couplage de la molécule antioxydante au nucléoside est de préférence réalisé sur un premier atome d'oxygène du nucléoside choisi parmi l'atome d'oxygène situé en position 5' du motif ribose ou désoxyribose et l'atome d'oxygène en position 3' du motif ribose ou désoxyribose.

Dans une telle configuration, préférentiellement :
- un groupement lipidant est porté par un deuxième atome d'oxygène du nucléoside, différent dudit premier atome d'oxygène, choisi parmi l'atome d'oxygène situé en position 5' du motif ribose ou désoxyribose et l'atome d'oxygène en position 3' du motif ribose ou désoxyribose,
- et/ou un groupement lipidant est porté par un atome d'azote d'un groupement amine primaire ou secondaire de la base nucléique du nucléoside.

Dans des modes de réalisation particuliers de l'invention, le nucléoside présente la formule générale (I) : 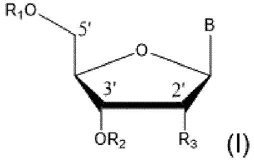 dans laquelle
B représente ladite base nucléique, éventuellement substituée au niveau d'un groupement amine par un groupement lipidant, chacun des groupements amines primaires éventuels de cette base nucléique non impliqué dans une liaison à la molécule antioxydante ou à un groupement lipidant étant substitué par un groupement protecteur d'une fonction amine primaire tel que défini ci-avant, et
- soit R₁ représente la liaison à la molécule antioxydante, et
   R₂ représente ledit groupement lipidant,
   R₃ représente H ou OR₄ où R₄ représente ledit groupement lipidant ou un groupement protecteur d'une fonction hydroxyle tel que défini ci-avant,
   ou R₂ et R₄ forment ensemble, avec les atomes d'oxygène auxquels ils sont rattachés, un groupement acétal comprenant une ou plusieurs chaines hydrocarbonée(s) linéaire(s), ramifiée(s) et/ou cycliques, en C1-C25, saturée(s) ou insaturée(s), aromatiques ou non, éventuellement substituées, éventuellement interrompues par un ou plusieurs hétéroatomes et/ou par un ou plusieurs groupements comprenant au moins un hétéroatome ou au moins un groupement comprenant au moins un hétéroatome, à l'exclusion des groupements hydroxyle, amine primaire, amine secondaire, phosphate et thiol, et pouvant comporter un cycle ou plusieurs cycles, le cas échéant un ou plusieurs hétérocycles comprenant un ou plusieurs hétéroatomes, les cycles étant optionnellement condensés,
- soit R₁ représente la liaison à ladite molécule antioxydante, et
   R₂ représente un groupement lipidant ou un groupement protecteur d'une fonction hydroxyle tel que défini ci-avant,
   R₃ représente OR₄ où R₄ représente un groupement lipidant,
- soit R₂ représente la liaison à la molécule antioxydante, et
   R₁ représente un groupement lipidant,
   R₃ représente H ou OR₄ où R₄ représente un groupement lipidant ou un groupement protecteur d'une fonction hydroxyle tel que défini ci-avant,
- soit R₂ représente la liaison à ladite molécule antioxydante, et
   R₁ représente un groupement lipidant ou un groupement protecteur d'une fonction hydroxyle tel que défini ci-avant,
   R₃ représente OR₄ où R₄ représente un groupement lipidant.

Dans des modes de réalisation dans lesquels R₁ représente la liaison à la molécule antioxydante et R₂ et R₄ forment ensemble, avec les atomes d'oxygène auxquels ils sont rattachés, un groupement acétal, ce dernier répond de préférence à la formule (VII) : 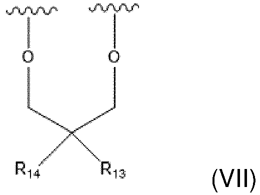
dans laquelle R₁₃ et R₁₄, identiques ou différents, peuvent représenter chacun un atome d'hydrogène, un groupement alkyle en C1-C25, par exemple en C1-C6, par exemple un groupement méthyle, ou un groupement oxyalkyle en C1-C25, par exemple en C1-C6,
ou R₁₃ et R₁₄ peuvent former ensemble, avec l'atome de carbone auquel ils sont rattachés, un groupement cycloalkyle en C3-C25, par exemple en C3-C6.

Dans des modes de réalisation particuliers de l'invention, la molécule antioxydante est couplée de manière covalente au nucléoside par l'intermédiaire d'un bras espaceur. Ce bras espaceur peut contenir au moins un groupe ester carboxylique. Il peut également ou autrement contenir au moins un groupement carbonyle lié à l'atome d'oxygène en position 5' du motif ribose ou désoxyribose ou à l'atome d'oxygène en position 3' du motif ribose ou désoxyribose du nucléoside ou à un atome d'oxygène de la molécule antioxydante, de sorte à former un groupement ester carboxylique avec cet atome d'oxygène.

Le bras espaceur est préférentiellement dépourvu de liaison du type phosphoramidate ou phosphodiester, ainsi que de liaison disulfure.

La liaison de la molécule antioxydante au nucléoside, via le bras espaceur, comporte de préférence un ou deux groupes ester carboxylique.

Préférentiellement, la molécule antioxydante est couplée de manière covalente au nucléoside par l'intermédiaire d'un bras espaceur de formule générale (II) : 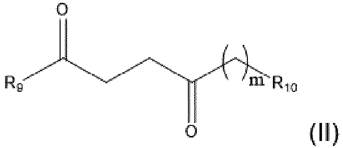 dans laquelle R₉ représente la molécule antioxydante et R₁₀ représente le nucléoside, et
- m est égal à 0 et la liaison de la molécule antioxydante au bras espaceur est portée par un atome d'oxygène de la molécule antioxydante et/ou la liaison du nucléoside au bras espaceur est portée par un atome d'oxygène du nucléoside,
- ou m est égal à 1 et la liaison de la molécule antioxydante au bras espaceur est portée par un atome d'oxygène de la molécule antioxydante.

La molécule antioxydante pharmaceutiquement active de la prodrogue selon l'invention est de préférence choisie parmi l'α-tocophérol, le β-tocophérol, le γ-tocophérol, l'α-tocotriénol, le β-tocotriénol, le γ-tocotriénol, le δ-tocotriénol, le fenchol, le resvératrol, l'acide lipoïque, le rétinol, le rétinal, l'acide rétinoïque et l'acide ascorbique.

Dans des variantes de l'invention, le composé actif répond à la formule générale (III) : 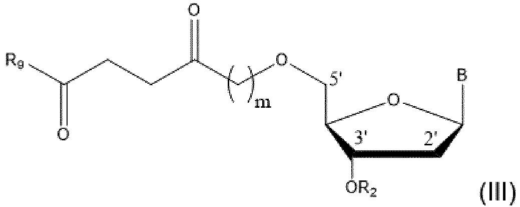 dans laquelle
B représente la base nucléique du nucléoside, chacun des groupements amines primaires éventuels de cette base nucléique non impliqué dans une liaison à ladite molécule antioxydante ou à un groupement lipidant étant substitué par un groupement protecteur d'une fonction amine primaire tel que défini ci-avant, par exemple par un reste benzyle, la base nucléique étant en particulier la thymine, R₂ représente le groupement lipidant, par exemple un reste benzyle,
R₉ représente la molécule antioxydante, optionnellement liée au bras espaceur au niveau d'un de ses atomes d'oxygène, lorsqu'elle comporte un ou plusieurs atomes d'oxygène,
et m est égal à 0 ou 1, m étant obligatoirement égal à 0 si la liaison de la molécule antioxydante au bras espaceur n'est pas portée par un atome d'oxygène de la molécule antioxydante.

Dans d'autres variantes de l'invention, le composé actif répond à la formule générale (III') : 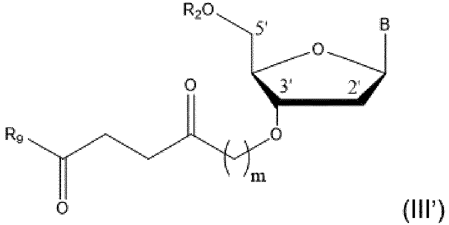 dans laquelle
B représente la base nucléique du nucléoside, chacun des groupements amines primaires éventuels de cette base nucléique non impliqué dans une liaison à ladite molécule antioxydante ou à un groupement lipidant étant substitué par un groupement protecteur d'une fonction amine primaire tel que défini ci-avant, par exemple par un reste benzyle, la base nucléique étant en particulier la thymine, R₂ représente le groupement lipidant, par exemple un reste benzyle,
R₉ représente la molécule antioxydante, optionnellement liée au bras espaceur au niveau d'un de ses atomes d'oxygène, lorsqu'elle comporte un ou plusieurs atomes d'oxygène,
et m est égal à 0 ou 1, m étant obligatoirement égal à 0 si la liaison de la molécule antioxydante au bras espaceur n'est pas portée par un atome d'oxygène de la molécule antioxydante.

Des composés actifs d'un intérêt tout particulier conformes à l'invention répondent aux formules générales suivantes :
a/ 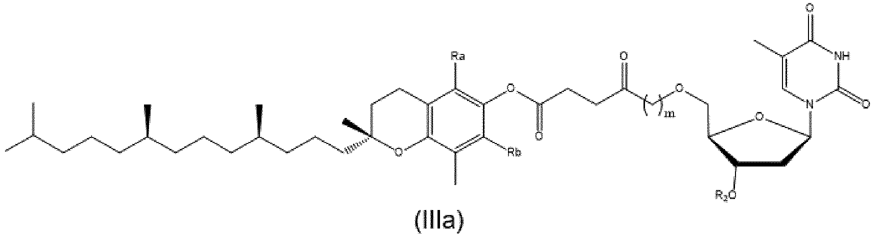 dans laquelle
   m est égal à 0 ou 1,
   Ra et Rb, identiques ou différents, représentent chacun un atome d'hydrogène ou un groupement méthyle,
   et R₂ représente le groupement lipidant, par exemple un reste benzyle ou un groupement -CO-R₁₁ où R₁₁ représente une chaine hydrocarbonée linéaire ou ramifiée, saturée ou insaturée en C1-C25, de préférence en C4-C25 et préférentiellement en C10-C25,
   la molécule antioxydante et le nucléoside dans ce composé actif étant respectivement un isomère du tocophérol et la thymidine ;
b/ 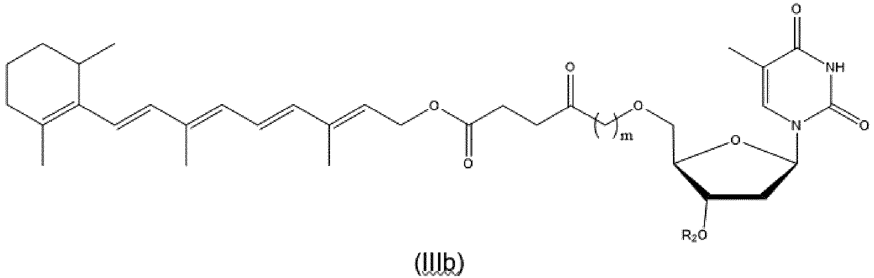 dans laquelle
   m est égal à 0 ou 1,
   et R₂ représente le groupement lipidant, par exemple un reste benzyle ou un groupement -CO-R₁₁ où R₁₁ représente une chaine hydrocarbonée linéaire ou ramifiée, saturée ou insaturée en C1-C25, de préférence en C4-C25 et préférentiellement en C10-C25,
   la molécule antioxydante et le nucléoside dans ce composé actif étant respectivement le rétinol et la thymidine ;
c/ 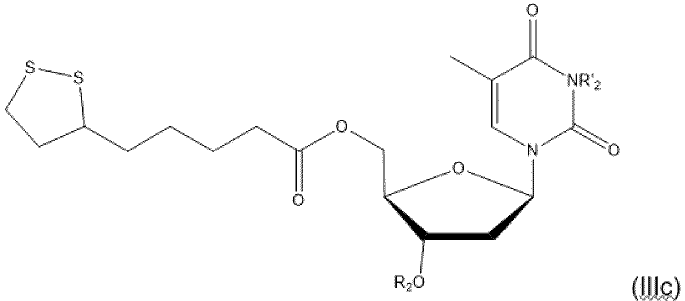
   dans laquelle R₂ et R'₂, identiques ou différents, représentent chacun un groupement lipidant, par exemple un reste benzyle ou un groupement -CO-R₁₁ où R₁₁ représente une chaine hydrocarbonée linéaire ou ramifiée, saturée ou insaturée en C1-C25, de préférence en C4-C25 et préférentiellement en C10-C25,
   la molécule antioxydante et le nucléoside dans ce composé actif étant respectivement l'acide lipoïque et la thymidine ;
d/ 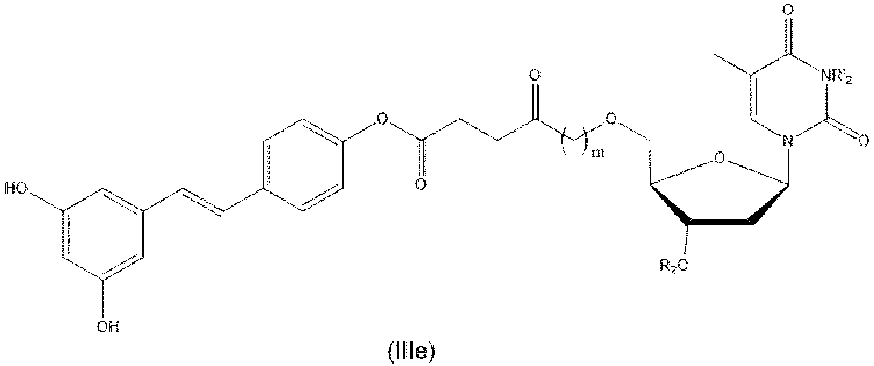 dans laquelle
   m est égal à 0 ou 1,
   et R₂ et R'₂, identiques ou différents, représentent chacun un groupement lipidant, par exemple un reste benzyle ou un groupement -CO-R₁₁ où R₁₁ représente une chaine hydrocarbonée linéaire ou ramifiée, saturée ou insaturée en C1-C25, de préférence en C4-C25 et préférentiellement en C10-C25,
   la molécule antioxydante et le nucléoside dans ce composé actif étant respectivement le resvératrol et la thymidine ;
e/ 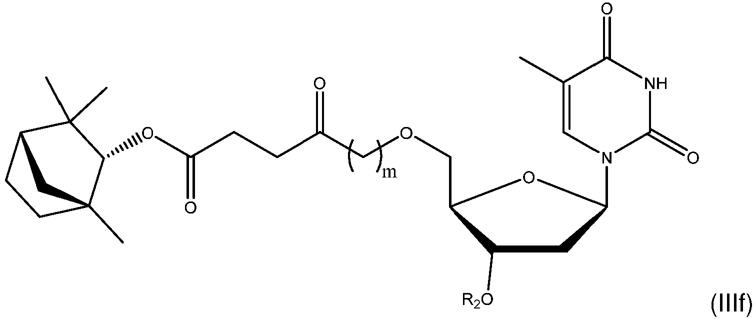 dans laquelle
   m est égal à 0 ou 1,
   et R₂ représente un groupement lipidant, par exemple un reste benzyle ou un groupement -CO-R₁₁ où R₁₁ représente une chaine hydrocarbonée linéaire ou ramifiée, saturée ou insaturée en C1-C25, de préférence en C4-C25 et préférentiellement en C10-C25,
   la molécule antioxydante et le nucléoside dans ce composé actif étant respectivement le fenchol et la thymidine.

En particulier, il a été montré par l'art antérieur, notamment illustré par la publication de Razazan et al., Frontiers in Aging Neuroscience, 2021, 13, Article 735933, un effet d'activation du récepteur d'acide gras libre 2 par la molécule antioxydante naturelle qu'est le fenchol, suggérant un rôle de cette dernière sur la progression de la maladie d'Alzheimer.

La prodrogue selon l'invention peut être préparée par toute méthode de synthèse chimique classique en elle-même.

Elle peut notamment être préparée par un procédé comprenant une étape de réaction entre un dérivé du nucléoside lipidisé fonctionnalisé de sorte à présenter une fonction aldéhyde libre et un dérivé de la molécule antioxydante fonctionnalisée de sorte à présenter une fonction carbonylvinyle, selon la réaction de Stetter, résultant en la formation de liaisons carbone-carbone par addition 1,4. Cette réaction d'addition peut être catalysée par un carbène N hétérocyclique, par exemple par le sel d'azolium bromure de 3-éthyl-5-(2-hydroxyéthyl)-4-méthylthiazolium. Elle peut notamment être réalisée selon une ou plusieurs, de préférence l'ensemble, des caractéristiques ci-après :
- en présence d'une base, telle que par exemple le 1,8-diazabicyclo[5.4.0]undéc-7-ène (DBU),
- dans un solvant polaire aprotique, tel que le tétrahydrofurane, ou le diméthylformamide, ou dans un solvant polaire protique tel que l'eau,
- à une température comprise entre 40 et 120 C, par exemple d'environ 75 °C,
- et/ou pendant 12 à 60 heures, par exemple environ 48 heures.

Le nucléoside lipidisé, son dérivé à fonction aldéhyde libre, et le dérivé de la molécule antioxydante à fonction carbonylvinyle peuvent être préparés de toute manière classique en soi.

Alternativement, la prodrogue selon l'invention peut être préparée par un procédé de synthèse comprenant une étape d'estérification d'un dérivé du nucléoside lipidisé fonctionnalisé de sorte à présenter une fonction acide carboxylique libre par la molécule antioxydante présentant une fonction hydroxyle libre, cette réaction d'estérification pouvant être réalisée de toute manière classique en elle-même.

Un autre aspect de l'invention concerne une composition pharmaceutique contenant, en tant que principe actif, une prodrogue selon l'invention dans un véhicule pharmaceutiquement acceptable.

On entend dans la présente description, par « véhicule pharmaceutiquement acceptable », tout véhicule utile pour la préparation d'une composition pharmaceutique et qui est généralement sûr, non toxique et ni biologiquement ni autrement indésirable pour le sujet à traiter, en particulier pour les mammifères et notamment les humains.

La composition pharmaceutique selon l'invention peut être formulée sous toute forme galénique, notamment sous une forme adaptée à une administration par voie orale, parentérale, intranasale, rectale, pulmonaire ou topique.

Préférentiellement, elle se présente sous une forme convenant à une administration par application topique, en particulier sur la peau et/ou les muqueuses, à une administration par voie parentérale, notamment par injection intraveineuse, ou à une administration par voie orale.

La composition pharmaceutique selon l'invention se présente préférentiellement sous forme d'une émulsion comprenant une phase lipophile et une phase aqueuse, la prodrogue étant contenue dans la phase lipophile. La prodrogue consiste alors en ledit composé actif ou un de ses sels solubles dans l'huile.

Préférentiellement, il s'agit d'une nanoémulsion, ou émulsion submicronique, c'est-à-dire d'une émulsion dans laquelle la taille moyenne des globules de la phase dispersée dans l'autre phase est inférieure à 300 nm, notamment comprise entre 10 et 250 nm.

Elle peut le cas échéant être gélifiée, par incorporation d'un ou plusieurs agent(s) gélifiant(s), classiques en eux-mêmes, par exemple choisis parmi les copolymères ramifiés d'oxyde d'éthylène et de polypropylène (poloxamères), les carbomères, les dérivés de la cellulose tels que l'hydroxyéthylcellulose, l'éthylcellulose et la méthylcellulose, etc. Elle consiste alors en une émulsion gélifiée, de préférence une nanoémulsion gélifiée, pouvant dans ce dernier cas être qualifiée de « nanoémulgel ». Le ou les agent(s) gélifiant(s) sont de préférence contenus dans la phase aqueuse de la composition.

La composition selon l'invention se présente de préférence sous forme d'une émulsion huile-dans-eau, qui présente notamment les avantages d'une meilleure absorption dans l'organisme, et d'une meilleure tolérance par les sujets traités lorsqu'elle est administrée par voie intraveineuse.

Ceci n'exclut pas pour autant les formes d'émulsion eau-dans-huile, ou d'émulsions multiples, par exemple eau-dans-huile-dans-eau, qui entrent également dans le cadre de l'invention, ou encore les formes SEDDS (pour l'anglais Self-Emulsifying Drug Delivery System, système de délivrance de drogue auto-émulsifiant).

Dans des modes de réalisation particuliers de l'invention, la composition comprend, en poids par rapport au poids total de la composition, 1 à 35 % de la phase lipophile, préférentiellement 10 à 30 % de la phase lipophile, par exemple environ 20 % de la phase lipophile.

L'ensemble des constituants de la composition selon l'invention sont bien entendus choisis pour être biocompatibles, c'est-à-dire compatibles avec une administration à un individu, notamment un mammifère et en particulier un humain.

La phase lipophile peut comprendre toute huile biocompatible classique en elle-même dans le domaine de la formulation galénique, ou mélange de telles huiles. Elle peut par exemple comprendre un ou plusieurs des ingrédients suivants : des acides gras et esters d'acide gras comprenant des acides carboxyliques de différentes longueur de chaine, saturés ou insaturés, comme l'acide arachidique, caprique, caprylique caproïque, myristique, palmitique, stéarique, laurique, oléique, linoléique, linolénique ou l'acide docosahexaénoïque, des acides gras estérifiés avec le glycérol formant des mono-,di- ou triglycérides, qui peuvent être synthétiques ou provenir de sources naturelles comme par exemple l'huile de soja, l'huile d'olive, l'huile de sésame, l'huile de coton, l'huile de ricin, l'huile d'œillette, l'huile de coprah, l'huile de noix de coco, l'huile de graines de lin, l'huile de tournesol, l'huile de triglycérides d'acides gras saturés en C6-C12, et les huiles de poissons, les produits commercialisés sous les noms Capmul^{®} MCM, Captex^{®} 300, Miglyol^{®} 812, le monooléate de glycéryle, la triacétine, les monoglycérides acétylés, la tristéarine, le béhénate de glycéryle, et les esters d'acide diacétyl tartarique et monoglycérides, etc.

La composition pharmaceutique selon l'invention contient en outre de préférence un agent émulsionnant, qui peut être de tout type classique en lui-même, notamment de type ionique ou non-ionique, ou un mélange de tels agents émulsionnants.

Le ou les agent(s) émulsionnant(s) peuvent par exemple être choisis parmi :
- les agents de surface phospholipides d'origine naturelle ou synthétique tels que les lécithines d'œuf ou de soja, les phospholipides pégylés ;
- les autres esters gras, tels que le myristate de myristyle, le palmitate d'isopropyle, le benzoate de benzyle, les esters de sorbitan (SPAN) tels que le laurate, palmitate, stéarate oléate et trioléate de sorbitan ;
- les esters de sorbitan polyhydroxyéthylénés ou polysorbates, comme les polysorbates 20, 60, 80 ou les sucroesters ;
- les glycérides conjugués à d'autres espèces, comme le polyéthylène glycol, par exemple les produits commercialisés sous les noms Labrasol^{®}, Labrafac^{®}, Cremophor EL^{®} ;
- les alcools gras tels que l'alcool stéarique, laurique ou benzylique ;
- ou l'un quelconque de leurs mélanges.

La concentration totale d'agent(s) émulsionnant(s) dans la composition est de préférence comprise entre 0,5 et 15 % en poids, de préférence entre 0,5 et 10 % en poids, préférentiellement entre 1 et 7,5 % en poids, et préférentiellement encore entre 1 et 5 % en poids, par rapport au poids total de la composition.

A titre d'exemple, lorsque l'agent émulsionnant est une lécithine, telle que la lécithine d'œuf E80, la concentration de cet agent émulsionnant dans la composition est de préférence comprise entre 1 et 2,5 % en poids, par exemple d'environ 1,5 % en poids. Lorsque l'agent émulsionnant est un polysorbate, tel que le polysorbate 80, la concentration de cet agent émulsionnant dans la composition est de préférence comprise entre 1 et 5 % en poids, par exemple d'environ 2,5 % en poids.

La composition pharmaceutique selon l'invention, sous forme d'émulsion, en particulier d'émulsion huile-dans-eau, et notamment de nanoémulsion, constitue avantageusement un système particulièrement efficace pour la délivrance de la prodrogue selon l'invention. Il a été découvert par les présents inventeurs qu'elle favorise plus encore le passage des membranes biologiques par la molécule antioxydante.

Ce système permet en outre de protéger la prodrogue des hydrolyses enzymatiques digestives avant l'absorption dans les membranes biologiques, ce qui s'avère tout à fait avantageux pour une administration par voie orale.

Formulée au sein d'un tel système de délivrance, la molécule antioxydante entrant dans la constitution de la prodrogue selon l'invention bénéficie à la fois du caractère promoteur d'absorption du nucléoside lipidisé selon l'invention, et de celui du système d'émulsion lipidique, notamment de nanoémulsion lipidique. Ceci lui permet notamment de franchir la barrière hémato-céphalique, ce qui la rend particulièrement appropriée pour le traitement des maladies du cerveau.

Elle est en outre libérée de manière doublement retardée dans l'organisme, après libération hors des globules de phase lipophile, puis activation par séparation du nucléoside lipidisé, par clivage de la liaison ester carboxylique par les estérases présentes de manière ubiquitaire dans l'organisme.

La composition pharmaceutique selon l'invention peut comprendre, exprimée en % en poids par rapport au poids total de la composition, une quantité comprise entre 0,1 et 10 %, de préférence comprise entre 0,1 et 5 %, préférentiellement comprise entre 1 et 5 %, ou encore entre 2 et 5 %, et par exemple environ 2 % à 3 %, de la prodrogue selon l'invention.

La concentration de la prodrogue selon l'invention dans la phase lipophile de la composition, la prodrogue consistant en ledit composé actif ou un de ses sels solubles dans l'huile, est quant à elle préférentiellement comprise entre 0,01 et 50 %, de préférence entre 0,1 et 30 %, préférentiellement entre 0,1 et 20 %, et préférentiellement encore entre 0,1 et 10 %, en poids par rapport au poids total de la phase lipophile.

Des compositions pharmaceutiques particulièrement préférées selon l'invention, sous forme d'émulsion comprenant une phase lipophile et une phase aqueuse, répondent à l'une ou plusieurs des caractéristiques suivantes, selon toute combinaison possible de ces caractéristiques, et notamment à l'ensemble de ces caractéristiques :
- elle consiste en une nanoémulsion, de préférence de type huile-dans-eau,
- elle comprend de 0,1 à 10 % en poids, notamment 2 à 5 % en poids, et par exemple 3 % en poids, par rapport au poids total de la composition, de la prodrogue selon l'invention,
- la prodrogue est solubilisée dans un véhicule lipophile, comprenant par exemple, ou constitué par, le produit connu sous le nom Miglyol^{®} 812,
- la phase lipophile représente 10 à 30 %, en poids, par exemple 20 % en poids, du poids total de la composition,
- elle contient au moins un agent émulsionnant choisi parmi les lécithines d'œuf et le polysorbate 80, ou l'un quelconque de leurs mélanges,
- et/ou la quantité d'agent(s) émulsionnant(s) dans la composition est comprise entre 1 et 7,5 % en poids, de préférence entre 2 et 7,5 % en poids, et préférentiellement entre 3 et 5 % en poids, par rapport au poids total de la composition.

Dans tous les cas, la quantité d'eau dans la composition est ajustée en fonction de celle des autres constituants, pour obtenir une quantité pondérale totale des constituants égale à 100 %.

La composition pharmaceutique selon l'invention, sous forme de nanoémulsion, se caractérise notamment par un aspect blanc-laiteux et, de préférence :
- une répartition granulométrique gaussienne avec un diamètre moyen, mesuré par diffusion dynamique de la lumière (DLS) ou diffusion quasi élastique de la lumière (QELS), inférieur à 300 nm, préférentiellement compris entre 10 et 250 nm,
- un indice de polydispersité (PDI) mesuré par DLS inférieur à 0,25
- et/ou un potentiel zeta, mesuré par diffusion électrophorétique de la lumière (ELS) de l'ordre de -35 mV.

Ses caractéristiques macroscopiques et granulométriques sont avantageusement stables dans le temps, sur une durée aussi longue que plusieurs semaines.

La composition pharmaceutique selon l'invention peut comprendre, en tant que principe actif, uniquement la prodrogue selon l'invention. Elle peut autrement contenir cette prodrogue en mélange avec une ou plusieurs autres substances pharmaceutiquement actives, agissant ou non en synergie avec la molécule antioxydante de la prodrogue selon l'invention.

Dans des modes de réalisation particuliers de l'invention, la composition pharmaceutique contient en outre une molécule antioxydante pharmaceutiquement active sous forme libre, c'est-à-dire non conjuguée à une autre molécule. Cette molécule antioxydante sous forme libre peut être identique à celle entrant dans la constitution de la prodrogue selon l'invention, ou être différente de cette dernière. Elle peut être présente dans la composition pharmaceutique selon l'invention telle quelle, ou le cas échéant sous forme d'un de ses sels solubles dans l'huile pharmaceutiquement acceptables.

A titre d'exemple, la molécule antioxydante sous forme libre peut être choisie parmi l'acide α-lipoïque, le fenchol, le resvératrol, les vitamines liposolubles comme la vitamine A, le rétinol, le ß-carotène et la vitamine E et ses dérivés, incluant tous les tocophérols et tocotriénols et leurs dérivés comme le succinate de vitamine E, l'acétate de vitamine E, et le succinate de vitamine E polyéthylène glycol (TPGS), ou l'un quelconque de leurs mélanges.

Un tel mode de réalisation permet notamment, tout à fait avantageusement, de moduler l'apport de la molécule antioxydante dans l'organisme, en combinant une libération immédiate de la molécule antioxydante contenue sous forme libre dans la composition, et une libération retardée de la molécule antioxydante entrant dans la constitution de la prodrogue selon l'invention, par l'hydrolyse *in vivo* de cette dernière, au niveau de la liaison ester carboxylique, par les estérases présentes dans l'organisme.

La composition pharmaceutique selon l'invention peut en outre contenir, en plus des constituants définis ci-avant, tout excipient ou additif pharmaceutiquement acceptable, ou mélange de tels excipients et/ou additifs.

On entend, par « pharmaceutiquement acceptable », le fait que l'excipient ou l'additif ne provoque aucun effet adverse, allergique ou autre réaction indésirable quand il est administré à un sujet, notamment à un mammifère, en particulier à un humain.

Un tel excipient ou additif peut être un diluant, un adjuvant, notamment lipidique, ou toute autre substance classique en elle-même pour la formulation des médicaments, telle qu'un agent de surface, un agent conservateur, un agent isotonisant, un agent d'ajustement de l'osmolalité et/ou du pH, notamment de l'hydroxyde de sodium, du glycérol, etc., La phase aqueuse peut notamment contenir un ou plusieurs co-solvants organiques, par exemple choisis chacun parmi l'éthanol, le polyéthylène glycol, le propylène glycol, le glycérol, les macrogols ou les polyoxyéthylène-glycols, par exemple le PEG 200, le PEG 300 ou le PEG 400.

La composition pharmaceutique selon l'invention peut en outre contenir un ou plusieurs ligands de ciblage tel qu'un lipide pégylé (phospholipide), un anticorps, un peptide, un aptamère, etc.

La composition pharmaceutique selon l'invention peut être conditionnée en récipient multidoses, ou bien sous forme de doses unitaires contenant chacune une quantité thérapeutiquement efficace de la prodrogue selon l'invention. La concentration de la prodrogue dans chaque dose de la composition pharmaceutique selon l'invention est de préférence choisie pour délivrer au sujet, à chaque administration, une quantité de prodrogue qui est efficace pour obtenir la réponse thérapeutique désirée.

La composition pharmaceutique selon l'invention peut optionnellement être stérile, de préférence à un niveau d'assurance de stérilité (NAS) inférieur ou égal à 10⁻⁶ selon la pharmacopée européenne 10.0.

La composition pharmaceutique selon l'invention peut être préparée par tout procédé classique en lui-même.

Lorsqu'elle se présente sous forme d'émulsion, elle est de préférence préparée par un procédé comprenant une étape d'inversion de phase.

A titre d'exemple, un procédé de préparation d'une composition pharmaceutique selon l'invention comprend les étapes successives suivantes :
- la solubilisation de la prodrogue selon l'invention dans une phase lipophile, ladite phase lipophile pouvant contenir une huile, éventuellement un agent de surface lipophile et optionnellement d'autres adjuvants, notamment lipidiques, par exemple de l'α-tocophérol sous forme libre et/ou un lipide pégylé ;
- la préparation d'une phase aqueuse comprenant au moins un agent émulsionnant, et éventuellement un ou des agents permettant l'ajustement de l'osmolalité et du pH ;
- le chauffage, séparément, de chacune de ces deux phases, à une température par exemple comprise entre 70 et 80 °C ;
- le mélange par inversion de phase en introduisant la phase aqueuse dans la phase lipophile à très haute vitesse, notamment au moyen d'un disperseur Ultra-Turrax^{®}, de manière à former une émulsion grossière dont les globules lipidiques présentent un diamètre moyen supérieur à 2 µm ;
- la réduction de la taille des globules lipidiques pour obtenir une nanoémulsion au moyen d'un homogénéiseur haute pression, d'un microfluidiseur ou d'une sonde à ultrasons, afin d'obtenir que 100 % des globules lipidiques présentent une taille inférieure à 1 µm avec un diamètre moyen compris entre 140 et 300 nm ;
- et, le cas échéant, la stérilisation de la nanoémulsion ainsi obtenue, par exemple à l'autoclave à 121 °C pendant 15 minutes, ou par filtration stérilisante. Un autre aspect de l'invention concerne l'utilisation d'une prodrogue selon l'invention, ou d'une composition pharmaceutique selon l'invention, en tant que médicament, pour le traitement thérapeutique d'un sujet en ayant besoin, c'est-à-dire atteint ou susceptible d'être atteint par la maladie, notamment d'un mammifère, en particulier d'un humain.

On entend dans la présente description, par le terme « traitement », un traitement prophylactique et/ou curatif d'une maladie, comprenant la prévention de la maladie ou la prévention totale ou partielle d'un ou plusieurs des symptômes de la maladie et/ou la guérison totale ou partielle de la maladie et/ou la disparition totale ou partielle d'un ou plusieurs de ses symptômes.

La prodrogue selon l'invention ou la composition selon l'invention sont de préférence administrées au sujet dans une quantité thérapeutiquement efficace. Par "quantité thérapeutiquement efficace", on entend la quantité qui, lorsqu'elle est administrée à un sujet pour traiter la maladie, est suffisante pour assurer un tel traitement de la maladie.

La quantité thérapeutiquement efficace de la prodrogue selon l'invention dépend de nombreux facteurs, tels que la maladie particulière et sa gravité, l'âge, le poids, etc., du sujet à traiter, la molécule antioxydante utilisée, la voie et la forme d'administration, etc. La quantité thérapeutiquement efficace de la prodrogue selon l'invention sera déterminée par le médecin pour chaque cas individuel.

L'administration de la prodrogue ou de la composition pharmaceutique selon l'invention au sujet à traiter peut être réalisée par toute voie classique en elle-même, notamment par voie parentérale, par exemple par voie sous-cutanée, sous-durale, intraveineuse, intramusculaire, intrathécale, intrapéritonéale, intracérébrale, intra-artérielle ou intra-lésionnelle ; par voie intranasale ; par voie rectale ; par voie pulmonaire, par exemple par aérosol ou inhalation ; par voie orale ; ou par voie topique, par exemple sur la peau ou les muqueuses.

La détermination de la posologie d'administration de la prodrogue ou de la composition pharmaceutique selon l'invention relève des compétences du médecin. La prodrogue ou la composition pharmaceutique peut par exemple être administrée au sujet en ayant besoin une ou deux fois par jour, sur une longue période, à intervalles réguliers, ou de manière ciblée lors d'un épisode aigu de la maladie.

Dans des modes de mise en œuvre particuliers de l'invention, la prodrogue ou la composition pharmaceutique peuvent être utilisées pour le traitement des brûlures radiologiques ou chimiques, en particulier des brûlures provoquées par l'ypérite.

L'ypérite est un gaz de combat provoquant de graves brûlures, qui a été utilisé dans de nombreuses guerres, depuis la première guerre mondiale jusqu'à plus récemment le conflit Iran-Irak des années 1980. Massivement stocké dans les arsenaux de la Guerre froide, il reste à ce jour l'un des agents chimiques de guerre les plus redoutables car imposant une protection très contraignante et un traitement médical lourd. Les anciens champs de bataille restent pollués par des obus chimiques non explosés contenant des quantités importantes d'ypérite, par exemple sur les côtes de la mer du Nord.

La prodrogue et la composition pharmaceutique selon l'invention trouvent une application particulièrement avantageuse pour le traitement des brûlures radiologiques ou des brûlures chimiques, notamment causées par l'ypérite, tirant profit des propriétés antioxydantes et cicatrisantes de la molécule pharmaceutiquement active de la prodrogue selon l'invention. La composition pharmaceutique selon l'invention pourrait en particulier être avantageusement utilisée lors du désarmement des obus restant dans les anciens champs de bataille, et également en cas de risque d'attaque au Moyen-Orient.

Dans un tel domaine d'application, une administration de la prodrogue ou la composition pharmaceutique selon l'invention par voie topique, cutanée ou ophtalmique, s'avère particulièrement préférée. Des configurations de la composition pharmaceutique selon l'invention comprenant un agent gélifiant, en particulier dans la phase aqueuse lorsque la composition se présente sous forme d'une émulsion, sont particulièrement préférées pour des administrations par voie topique cutanée ou ophtalmique.

Dans d'autres modes de mise en œuvre particuliers de l'invention, la prodrogue ou la composition pharmaceutique selon l'invention sont utilisées pour le traitement d'une maladie neurodégénérative, notamment de la maladie de Parkinson, de la maladie d'Alzheimer, ou de toute autre maladie pour laquelle la cible pharmacologique est le système nerveux central.

De nombreuses maladies neurodégénératives, telles que la maladie de Parkinson, la maladie d'Alzheimer, etc., demeurent aujourd'hui incurables et les traitements symptomatiques développés présentent de nombreuses limites. Un problème physiologique majeur à l'élaboration d'un traitement chronique est notamment le franchissement de la Barrière Hémato-Encéphalique (BHE).

Il a été découvert par les présents inventeurs que la prodrogue selon l'invention, et la composition pharmaceutique qui la contient, d'autant plus sous forme de nanoémulsion lipidique, permettent avantageusement d'assurer une délivrance de la molécule antioxydante de la prodrogue au sein du système nerveux central, dans des cellules neuronales et les organites intracellulaires (lysosomes). Plus particulièrement, il a été découvert par les inventeurs que l'effet combiné du nucléoside lipidisé conjugué à la molécule antioxydante et de la forme de nanoémulsion lipidique permet de véhiculer la molécule antioxydante jusqu'au système nerveux central, la prodrogue selon l'invention étant alors capable de pénétrer dans les neurones humains, au sein desquels la molécule antioxydante est libérée par hydrolyse catalysée par les estérases présentes *in situ.* Une telle délivrance ciblée permet notamment avantageusement de retarder la progression de la maladie neurologique et de ses symptômes. On ne préjugera pas ici des mécanismes se produisant au niveau cellulaire sous-tendant l'obtention d'un tel résultat avantageux. On peut penser qu'y participerait le fait qu'en plus de la molécule antioxydante libérée, le nucléolipide issu de la prodrogue est alors capable d'acidifier les lysosomes, résultant en une restauration de la fonction lysosomale dégradée par la maladie.

Dans un tel contexte d'application au traitement des maladies dégénératives, la prodrogue ou la composition pharmaceutique selon l'invention sont de préférence administrées au sujet à traiter par une voie d'administration systémique, notamment par voie orale ou par voie parentérale, en particulier par voie intraveineuse.

La prodrogue ou la composition pharmaceutique selon l'invention peuvent autrement être utilisées pour le traitement de maladies ophtalmiques, telles que la DMLA, pour favoriser la cicatrisation cutanée, ou pour des applications cométiques, notamment pour le traitement cutané anti-âge.

La présente invention s'exprime également sous forme d'un procédé de traitement, prophylactique ou curatif, d'une maladie chez un sujet. Le sujet peut être, en particulier, un mammifère et préférentiellement un être humain. Ce procédé comprend l'administration, audit sujet en ayant besoin, d'une quantité thérapeutiquement efficace de la prodrogue ou de la composition pharmaceutique selon l'invention.

Ce procédé peut répondre à l'une ou plusieurs des caractéristiques décrites ci-avant en référence à l'utilisation de la prodrogue ou de la composition pharmaceutique selon l'invention en tant que médicament.

La présente invention s'exprime également dans les termes de l'utilisation, pour le traitement, prophylactique ou curatif, d'une maladie chez un sujet, ou pour la fabrication d'un médicament, d'une prodrogue ou d'une composition pharmaceutique selon l'invention.

Cette utilisation peut répondre à l'une ou plusieurs des caractéristiques décrites ci-avant en référence à la prodrogue et à la composition pharmaceutique selon l'invention et à leur utilisation.

La prodrogue et la composition pharmaceutique selon l'invention peuvent autrement être utilisées pour la fabrication de dispositifs médicaux, notamment pour l'infusion de prothèses, ce qui permet avantageusement la préservation des propriétés mécaniques et des performances de ces dernières lors de leur utilisation.

Les caractéristiques et avantages de l'invention apparaîtront plus clairement à la lumière des exemples de mise en œuvre ci-après, fournis à simple titre illustratif et nullement limitatifs de l'invention, avec l'appui des figures 1 à 12, dans lesquelles :
La figure 1 représente le schéma réactionnel de synthèse d'un dérivé de l'α-tocophérol portant une fonction carbonylvinyle.
La figure 2 représente le schéma de synthèse d'une prodrogue conforme à l'invention comprenant une molécule d'α-tocophérol liée au nucléoside thymidine portant un groupe lipidant (groupe benzyle sur l'oxygène en position 3' du motif désoxyribose).
La figure 3 représente le schéma de synthèse d'une prodrogue conforme à l'invention comprenant une molécule d'α-tocophérol liée au nucléoside thymidine portant un groupe lipidant (groupe méthyl-naphtyle sur l'oxygène en position 3' du motif désoxyribose).
La figure 4 représente le schéma de synthèse d'une prodrogue conforme à l'invention comprenant une molécule d'α-tocophérol liée au nucléoside thymidine portant un groupe lipidant (groupe benzyle sur l'oxygène en position 3' du motif désoxyribose).
La figure 5 représente des graphes montrant le % de viabilité cellulaire de cellules neuronales humaines non traitées (NT) ou après 24 h (en a/) ou 48 h (en b/) de traitement par une composition pharmaceutique sous forme de nanoémulsion conforme à l'invention (NE), contenant 2 % p/p de composé actif.
La figure 6 représente le schéma de synthèse d'une prodrogue conforme à l'invention comprenant une molécule de fenchol liée au nucléoside thymidine portant un groupe lipidant (groupe benzyle sur l'oxygène en position 3' du motif désoxyribose).
La figure 7 représente le schéma de synthèse d'un composé non conforme à l'invention comprenant une molécule d'α-tocophérol liée au nucléoside thymidine dépourvu de groupe lipidant ; TBDPS représente un groupement tertbutyldiphénylsilyl.
La figure 8 montre des dilutions dans le véhicule huileux Miglyol^{®} 812 de : a/ un composé conforme à l'invention comprenant une molécule d'α-tocophérol liée au nucléoside thymidine portant un groupe lipidant (groupe benzyle sur l'oxygène en position 3' du motif désoxyribose), dilué à 5 % p/p ; b/ un composé comparatif similaire mais dépourvu du groupe lipidant, dilué à 1 % p/p ; c/ le même composé comparatif dilué à 2 % p/p ; d/ le même composé comparatif dilué à 5 % p/p.
La figure 9 montre le spectre de RMN ¹H d'un composé conforme à l'invention comprenant une molécule d'α-tocophérol liée au nucléoside thymidine portant un groupe lipidant (groupe benzyle sur l'oxygène en position 3' du motif désoxyribose).
La figure 10 montre le spectre de RMN ¹H du composé de la figure 9 après 8 h d'irradiation UV.
La figure 11 montre un graphe en barres représentant le % d'intensité de fluorescence moyenne (MFI) par rapport à une condition contrôle non irradiée, obtenue en présence de l'indicateur de stress oxydatif CM-H₂DCFDA, pour des kératinocytes irradiés selon le spectre solaire après incubation pendant 30 min en présence d'une composition conforme à l'invention sous forme de nanoémulsion contenant 5 % p/p d'un composé actif selon l'invention, diluée au 1/1000^{ème} dans le milieu (« C4 ») ou en absence d'une telle composition (« CT »).

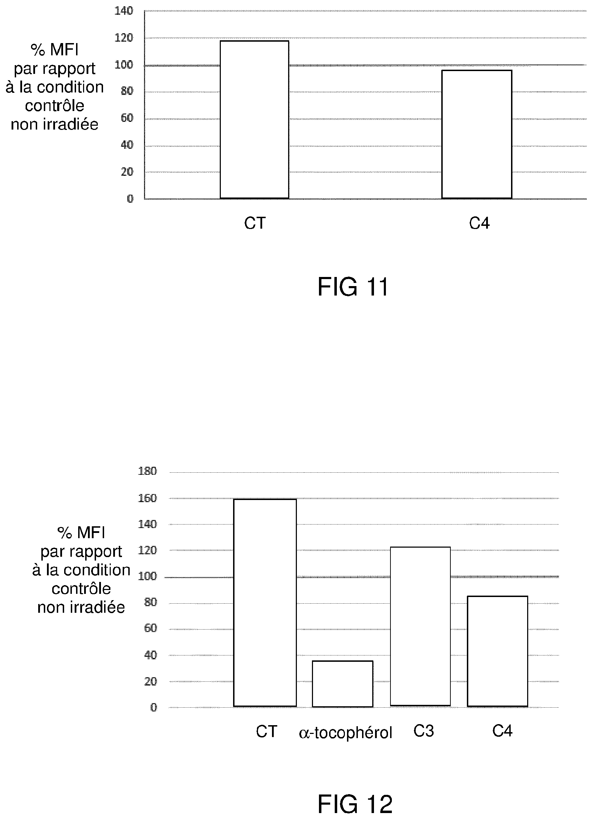

La figure 12 montre un graphe en barres représentant le % d'intensité de fluorescence moyenne (MFI) par rapport à une condition contrôle non irradiée, obtenue en présence de l'indicateur de stress oxydatif CM-H₂DCFDA, pour des kératinocytes irradiés selon le spectre solaire après incubation pendant 48 h en présence : d'une composition conforme à l'invention sous forme de nanoémulsion contenant 5 % p/p d'un composé actif selon l'invention, diluée au 1/1000^{ème} dans le milieu (« C4 ») ; d'une composition conforme à l'invention sous forme de nanoémulsion contenant 3 % p/p d'un composé actif selon l'invention, diluée au 1/1000^{ème} dans le milieu (« C3 ») ; d'α-tocophérol à 0,5 % p/v ; ou en absence de tout composé (« CT »).

### Exemple1

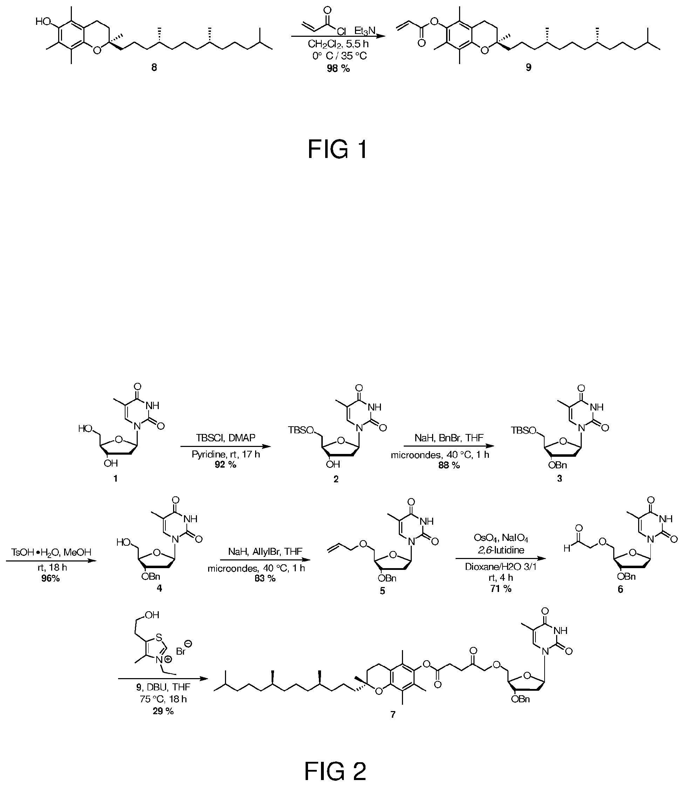

Un dérivé de l'α-tocophérol (composé 9) est obtenu à partir d'α-tocophérol 8 selon le schéma réactionnel montré sur la figure 1.

A cet effet, de la triéthylamine Et₃N (1,2 éq., 100 µL, 0,70 mmol) est ajoutée goutte à goutte à une solution d'a-tocophérol 8 (1,0 éq., 250 mg, 0,58 mmol) dans du dichlorométhane (0,1 M, 5,8 mL). A 0 °C, du chlorure d'acryloyle (2,0 éq., 94 µL, 1,16 mmol) est ajouté goute à goute. Le milieu réactionnel est agité pendant 2 h à 0 °C avant d'être agité pendant 3,5 h à 35 °C. Après refroidissement à température ambiante, le milieu est lavé 3 fois avec une solution saturée de NaHCO₃. La phase organique est récupérée, séchée sur Na₂SO₄, filtrée et concentrée sous pression réduite. Le brut réactionnel est purifié par chromatographie sur gel de silice (EtOAc/Pentane 1/99) pour obtenir le composé 9 (274,5 mg, 98 %, huile incolore).

Les données de caractérisation de ce composé sont en accord avec les données publiées dans la littérature (Xie et al., J. Am. Chem. Soc., 2020, 142, 16787-16794).

### Exemple 2

Un composé actif selon l'invention (Composé 7) est préparé selon le schéma réactionnel montré sur la figure 2, à partir du dérivé d'α-tocophérol 9 préparé à l'Exemple 1, et de thymidine 1.

A partir de thymidine 1, le composé 2 est obtenu comme décrit dans la publication de Maturano et al., Eur. J. Org. Chem., 2012, 721.

Dans un flacon à 0 °C, de l'hydrure de sodium NaH (dispersion à 60 % dans une huile, 280 mg, 7,01 mmol) est ajouté par portions dans une solution du composé 2 (1 éq., 1,00 g, 2,80 mmol) dans le tétrahydrofurane THF (0,1 g/mL, 10 mL). Le milieu réactionnel est activé par des microondes (CEM machine, 200 W) à 40 °C pendant 4,5 min. Du bromure de benzyle (2,5 éq., 833 µL, 7,01 mmol) est ajouté au milieu réactionnel qui est ensuite soumis à une deuxième activation aux microondes (CEM machine, 200) à 40 °C pendant 1 h. Après refroidissement à température ambiante, la réaction est arrêtée avec une solution saturée de chlorure d'ammonium NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 30/70) pour obtenir le composé 3 (1,013 g, 88 %, huile incolore), dont les données de caractérisation sont conformes avec celles publiées dans la littérature (Teste et al., Carbohydrate Research, 2008, 343, 1490).

De l'acide paratoluènesulfonique monohydraté (0,2 éq., 86 mg, 0,45 mmol) est ajouté à une solution du composé 3 (1,0 éq., 1,013g, 2,27 mmol) dans du méthanol (0,1 g/mL, 9 mL). Après 18 h d'agitation à température ambiante, le milieu réactionnel est concentré sous pression réduite. Le résidu est dissout dans du dichlorométhane. La phase organique est lavée avec une solution saturée de NaHCOs puis de la saumure. La phase organique est récupérée, séchée sur Na₂SO₄, filtrée et concentrée sous pression réduite. Le brut réactionnel est purifié par chromatographie sur gel de silice (MeOH/CH₂Cl₂ 5/95) pour obtenir le composé 4 (724 mg, 96 %, mousse blanche).
Rf = 0,25 (Pentane/EtOAc 30/70)
IR (ATR) vₘₐₓ (cm⁻¹) 3422, 3186, 3062, 3038, 2928, 1683, 1471, 1405, 1366, 1324, 1275, 1203, 1132, 1096, 1058, 960, 910, 787, 736, 699, 651, 606, 560, 492
RMN - ¹H (300 MHz, CDCl₃) δ (ppm) 8.99 (s, 1H), 7.42 - 7.29 (m, 6H), 6.14 (apparait t, *J* = 6.6, 7.5 Hz, 1H), 4.54 (AB système, *J =* 11.8 Hz, 2H), 4.33 - 4.26 (m, 1H), 4.20 - 4.13 (m, 1H), 3.93 (dd, J = 2.4, 11.7 Hz, 1H), 3.75 (dd, J = 3.0, 12 Hz, 1H), 2.48 - 2.28 (m, 2H), 1.90 (s, 3H)
RMN - ¹³C (75.5 MHz, CDCl₃) δ (ppm) 163.9, 150.5, 137.6, 137.2, 128.7, 128.1, 127.8, 111.2, 87.5, 85.3, 78.7, 71.8, 62.9, 37.3, 12.6
HRMS (ESI): calculé pour C₁₇H₂₀N₂O₅Na [M+Na]⁺ 355,12644 ; trouvé 355,1265. Dans un flacon à 0 °C, du NaH (dispersion à 60 % dans une huile, 248 mg, 6,20 mmol) est ajouté par portions dans une solution du composé 4 (1 éq., 825 mg, 2,48 mmol) dans le THF (0,1 g/mL, 8,3 mL). Le milieu réactionnel est activé par des microondes (CEM machine, 200 W) à 40 °C pendant 2 min. Le bromure d'allyle (2,5 éq., 536 µL, 6,20 mmol) est ajouté au milieu réactionnel qui est ensuite soumis à une deuxième activation aux microondes (CEM machine, 200) à 40 °C pendant 1 h. Après refroidissement à température ambiante, la réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 40/60 à 50/50) pour obtenir le composé 5 (766 mg, 83 %, huile incolore).
Rf = 0,37 (Pentane/EtOAc 50/50)
IR (ATR) vₘₐₓ (cm⁻¹) 3180, 3064, 3035, 2925, 2861, 1682, 1468, 1434, 1402, 1364, 1329, 1274, 1202, 1096, 1055, 1029, 960, 914, 884, 785, 733, 698, 670, 645, 608, 558, 491
RMN - ¹H (300 MHz, CDCl₃) δ (ppm) 9.28 - 9.07 (br. s, 1H), 7.63 (s, 1H), 7.40 - 7.27 (m, 5H), 6.39 (dd, *J =* 6.0, 7.8 Hz, 1H), 5.97 - 5.82 (m, 1H), 5.32 - 5.18 (m, 2H), 4.54 (dd, *J =* 12.0, 26.7 Hz, 2H), 4.28 - 4.21 (m, 2H), 4.06 - 4.01 (m, 2H), 3.74 (dd, *J =* 2.4, 10.8 Hz, 1H), 3.58 (dd, J = 2.1, 10.5 Hz, 1H), 2.47 (ddd, *J* = 1.8, 5.7, 12.9 Hz, 1H), 2.16 - 2.05 (m, 1H), 1.89 (d, *J =* 0.9 Hz, 3H)
RMN - ¹³C-NMR (75.5 MHz, CDCl₃) δ (ppm) 164.0, 150.5, 137.6, 136.0, 134.0, 128.6, 128.0, 127.7, 117.6, 110.9, 85.3, 84.1, 79.3, 77.4, 72.4, 71.5, 70.5, 38.1, 12.7
HRMS (ESI): calculé pour C₂₀H₂₄N₂O₅Na [M+Na]⁺ 395,15774 ; trouvé 395,1581. La 2,6-lutidine (2 éq., 713 µL, 5,62 mmol) est ajoutée à une solution du composé 5 (1 éq., 1,046 g, 2,81 mmol) dans un mélange Dioxane/H₂O (3/1, 0,1 M, 28 mL). Une solution de tétroxyde d'osmium (2,5 w% dans du tert-butanol, 0,03 éq., 851 µL, 0,084 mmol) et du périodate de sodium (3 éq., 2,14 g, 8,43 mmol) sont ajoutés successivement au milieu réactionnel. Le milieu réactionnel blanc est agité à température ambiante pendant 4 à 5 h. La réaction est arrêtée avec de l'eau. Le milieu est extrait 3 fois avec du dichlorométhane. Les phases organiques sont rassemblées, lavées avec une solution saturée de Na₂S₂O₅, séchées sur Na₂SO₄, filtrées et concentrées sous pression réduite. Le brut réactionnel est purifié par chromatographie sur gel de silice (Acétone/Toluène 30/70 à 40/60 avec quelques gouttes de Et₃N) pour obtenir le composé 6 (747 mg, 71 %, mousse blanche).
Rf = 0,28 (Toluène/Acétone 70/30)
IR (ATR) vmax (cm⁻¹) 3385, 3202, 3064, 3038, 2920, 2872, 1687, 1468, 1403, 1357, 1329, 1276, 1202, 1134, 1080, 962, 910, 773, 738, 700, 604, 560, 492 RMN - ¹H (300 MHz, CDCl₃) δ (ppm) 9.67 (s, 1H), 9.26 (br. s, 1H), 7.57 (s, 1H), 7.41 - 7.25 (m, 5H), 6.37 (dd, *J =* 6.0, 7.5 Hz, 1H), 4.55 (dd, *J =* 11.7, 28.8 Hz, 2H), 4.33 - 4.27 (m, 1H), 4.26 - 4.16 (m, 3H), 3.81 (dd, J = 2.7, 10.2 Hz, 1H), 3.67 (dd, *J =* 3.0, 10.5 Hz, 1H), 2.46 (ddd, J = 2.7, 6.0, 13.8 Hz, 1H), 2.22 - 2.09 (m, 1H), 1.88 (d, *J =* 0.9 Hz, 3H)
RMN - ¹³C (75.5 MHz, CDCl₃) δ (ppm) 198.0, 164.1, 150.6, 137.6, 136.0, 128.6, 128.6, 128.1, 127.8, 127.7, 111.2, 85.4, 83.6, 79.1, 77.4, 71.7, 71.6, 37.7, 12.6 HRMS (ESI): calculé pour C₁₉H₂₂N₂O₆Na [M+Na]⁺ 397,1370 ; trouvé 397,1368. Le sel d'azolium bromure de 3-éthyl-5-(2-hydroxyéthyl)-4-méthylthiazolium (0,3 éq., 52 mg, 0,210 mmol), préalablement séché sous vide pendant 1 jour à 45 °C est chargé dans un tube Schlenk et mis sous argon. Une solution du composé 6 (1,5 éq., 384 mg, 1,02 mmol) dans du THF (0,5 M, 2,1 mL) est ajoutée au tube Schlenk suivi par de 1,8-diazabicyclo[5.4.0]undéc-7-ène (DBU) distillée (0,3 éq., 31 µL, 0,21 mmol). Le milieu devient orange à la suite de la formation de l'intermédiaire de Breslow. Le dérivé d'α-tocophérol 9 (1,0 éq., 331 mg, 0,683 mmol) est ajouté au milieu qui est agité à 75 °C pendant 18 h. Après refroidissement à température ambiante, le milieu est concentré sous pression réduite. Le brut réactionnel est purifié par chromatographie sur gel de silice (Acétone/Toluène 20/80) pour obtenir le composé 7 (126 mg, 29 %, gomme beige) conforme à l'invention.
Rf = 0,49 (Acétone/Toluène 20/80)
IR (ATR) vₘₐₓ (cm⁻¹) 2920, 2852, 1719, 1690, 1457, 1423, 1369, 1330, 1276, 1252, 1208, 1157, 1136, 1082, 1056, 1008, 962, 936, 871, 780, 737, 699, 653, 611, 556, 493, 422
RMN - ¹H (300 MHz, CDCl₃) δ (ppm) 8.57 (brs, 1H), 7.66 (d, *J =* 1.5 Hz, 1H), 7.40 - 7.27 (m, 5 H), 6.38 (dd, *J =* 5.7, 8.1 Hz, 1H), 6.55 (dd, J = 13.5, 25.2 Hz, 2H), 4.35 - 4.18 (m, 4H), 3.78 (dd, *J* = 2.7, 10.5 Hz, 1H), 3.63 (dd, *J =* 3.0, 10.2 Hz, 1H), 3.02 - 2.93 (m, 2H), 2.79 - 2.69 (m, 2H), 2.62 - 2.52 (m, 2H), 2.46 (ddd, *J =* 2.4, 5.7, 13.5 Hz, 1H), 2.19 - 2.10 (m, 1H), 2.07 (s, 3H), 2.00 (s, 3H), 1.96 (s, 3H), 1.89 (s, 3H), 1.85 - 1.66 (m, 3H), 1.61 - 0.98 (m, 26H), 0.92 - 0.79 (m, 12H)
RMN - ¹³C (75.5 MHz, CDCl₃) δ (ppm) 205.3, 171.5, 164.0, 150.6, 149.6, 140.5, 137.6, 136.1, 128.6, 128.0, 127.8, 126.7, 124.9, 123.2, 117.5, 111.2, 85.3, 83.6, 79.3, 76.0, 75.2, 71.6, 71.6, 39.5, 37.7 - 37.4 (plusieurs C : CH, CH₂ aliphatiques), 33.2, 33.9, 32.8, 28.1, 27.4, 24.9, 24.5, 22.8, 22.7, 21.1, 20.7, 19.9, 19.8, 19.7, 13.1, 12.6, 12.2, 11.9
HRMS (ESI): calculé pour C₅₁H₇₅O₉ N₂ [M+H]⁺ 859,54671 ; trouvé 859,54671.

### Exemple 3

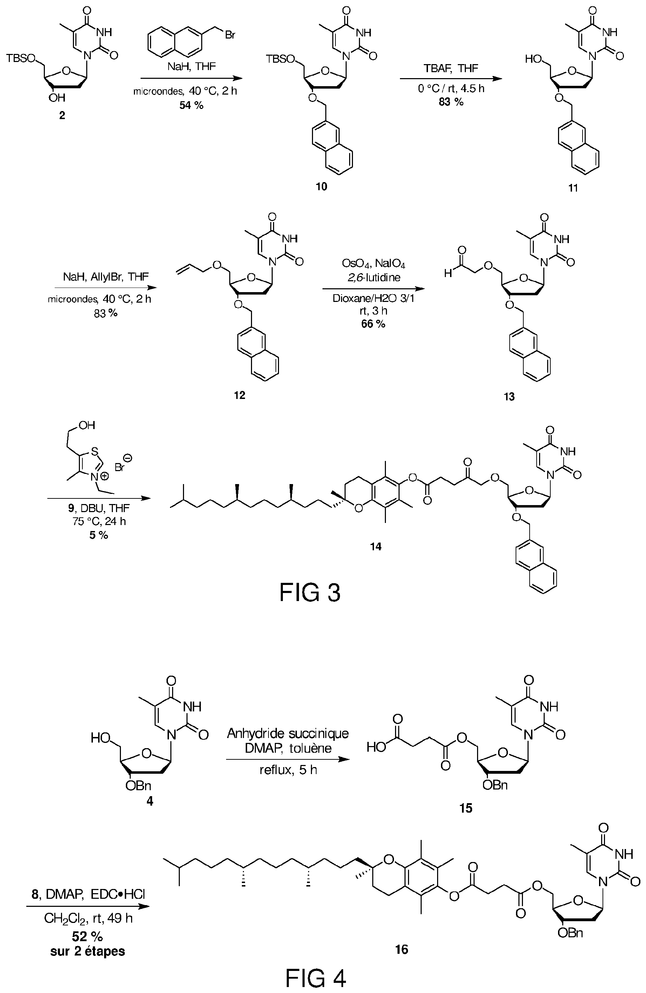

Un composé actif selon l'invention (Composé **14**) est préparé selon le schéma réactionnel montré sur la figure 3, à partir du dérivé d'α-tocophérol **9** préparé à l'Exemple 1, et du composé **2** dérivé de thymidine obtenu comme décrit dans l'Exemple 2.

Dans un flacon à 0 °C, du NaH (dispersion à 60 % dans une huile, 2,5 éq., 280 mg, 7,01 mmol) est ajouté par portions dans une solution du composé **2** (1 éq., 1,00 g, 2,80 mmol) dans le THF (0,1 g/mL, 10 mL). Le milieu réactionnel est activé par des microondes (CEM machine, 200 W) à 40 °C pendant 4,5 min. Le bromure de 2-méthylnaphtalène (2,5 éq., 1,55 g, 7,01 mmol) est ajouté au milieu réactionnel qui est ensuite soumis à une deuxième activation aux microondes (CEM machine, 200) à 40 °C pendant 2 h. Après refroidissement à température ambiante, la réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 30/70) pour obtenir le composé **10** (747,2 mg, 54 %, gomme incolore).

Rf = 0,32 (EtOAc/Pentane 30/70)
IR (ATR) vₘₐₓ (cm⁻¹) 2926, 2854, 1735, 1704, 1644, 1469, 1377, 1248, 1146, 1104, 998, 931, 781, 722, 619, 554, 496, 416.

RMN - ¹H (300 MHz, CDCl₃) δ (ppm) 9.56 (brs, 1H), 7.88 - 7.80 (m, 3H), 7.77 (s, 1H), 7.53 - 7.42 (m, 4H), 6.40 (dd, *J* = 5.7, 8.7 Hz, 1H), 4.70 (dd, *J =* 12.0, 30.9 Hz, 2H), 4.24 - 4.17 (m, 2H), 3.89 (dd, *J* = 2.4, 11.4 Hz, 1H), 3.72 (dd, *J =* 2.1, 11.4 Hz, 1H), 2.55 (dd, *J* = 5.7, 13.2 Hz, 1H), 2.04 - 1.95 (m, 1H), 1.92 (s, 3H), 0.87 (s, 9H), 0.04 (d, *J =* 8.4 Hz, 6H)
RMN - ¹³C (75.5 MHz, CDCl₃) δ (ppm) 164.2, 150.6, 135.5, 135.0, 133.3, 133.1, 128.5, 127.9, 127.8, 126.7, 126.3, 126.1, 125.7, 111.0, 85.2, 85.1, 79.0, 71.5, 63.7, 38.1, 25.9, 18.4, 12.6, - 5.3, - 5.5.

A 0 °C, une solution de fluorure de tertbutylammonium dans du THF (1,0 M, 1 éq., 4,52 mL, 4,52 mmol) est ajoutée goutte à goutte à une solution du composé **10** (1 éq., 2,25 g, 4,52 mmol) dans le THF (0,1 M, 45,2 mL). Le milieu est agité à température ambiante pendant 4,5 h. La réaction est arrêtée avec de l'eau puis le milieu est extrait 3 fois au dichlorométhane. Les phases organiques sont rassemblées, lavées avec de la saumure, séchées sur Na₂SO₄, filtrées et concentrées sous pression réduite. Le brut réactionnel est purifié par chromatographie sur gel de silice (CH₂Cl₂/MeOH 95/5) pour obtenir le composé **11** (1,43 g, 83 %, solide blanc).

Rf = 0,42 (EtOAc/Pentane 70/30)
IR (ATR) vₘₐₓ (cm⁻¹) 3503.6, 3166.8, 3065.2, 3023.7, 2921.6, 1709.0, 1659.9, 1508.0, 1467.8, 1398.2, 1369.2, 1346.4, 1325.1, 1281.6, 1255.5, 1225.5, 1185.9, 1058.8, 1012.0, 974.5, 955.9, 906.5, 882.6, 859.2, 785.8, 768.2, 752.3, 653.2, 635.7, 608.7, 568.2, 549.5, 497.3, 441.8, 407.5
RMN - ¹H (300 MHz, dmso-d₆) δ (ppm) 11.35 (brs, 1H), 7.96 - 7.84 (m, 4H), 7.72 (d, *J* = 1.5 Hz, 1H), 7.56 - 7.45 (m, 3H), 6.22 (dd, *J* = 5.7, 8.4 Hz, 1H), 5.21 - 5.09 (m, 1H), 4.76 - 4.66 (m, 2H), 4.27 - 4.20 (m, 1H), 4.11 - 4.06 (m, 1H), 3.69 - 3.55 (m, 2H), 2.34 (ddd, *J* = 2.4, 6.0, 13.8 Hz, 1H), 2.24 - 2.11 (m, 1H), 1.78 (d, *J =* 1.2 Hz, 3H)
RMN - ¹³C (75.5 MHz, dmso-d₆) δ (ppm) 163.8, 150.5, 136.0, 135.8, 132.8, 132.5, 127.9, 127.8, 127.6, 126.3, 126.0, 126.0, 125.9, 109.6, 84.8, 83.9, 79.1, 70.2, 61.6, 36.4, 12.3
HRMS (ESI): Calculé pour C₂₁H₂₂O₅N₂Na [M+Na]⁺ 405,14209 ; trouvé 405,1424. Dans un flacon à 0 °C, du NaH (dispersion à 60 % dans une huile, 2,5 éq., 174 mg, 4,35 mmol) est ajouté par portions dans une solution du composé **11** (1 éq., 669 mg, 1,74 mmol) dans le THF (0,1 g/mL, 6,7 mL). Le milieu réactionnel est activé par des microondes (CEM machine, 200 W) à 40 °C pendant 2 min. Du bromure d'allyle (2,5 éq., 376 µL, 4,35 mmol) est ajouté au milieu réactionnel qui est ensuite soumis à une deuxième activation aux microondes (CEM machine, 200) à 40 °C pendant 2 h. Après refroidissement à température ambiante, la réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 50/50) pour obtenir le composé **12** (607 mg, 83 %, solide blanc).

Rf = 0,40 (EtOAc/Pentane 50/50)
IR (ATR) vₘₐₓ (cm⁻¹) 2932, 2860, 1731, 1643, 1462, 1378, 1249, 1147, 1106, 781, 622, 552, 419.

RMN - ¹H (300 MHz, Acétone-d₆) δ (ppm) 9.94 (brs, 1H), 7.94 - 7.86 (m, 4H), 7.69 (d, *J =* 1.2 Hz, 1H), 7.57 - 7.46 (m, 3H), 6.38 (dd, J = 6.0, 8.4 Hz, 1H), 6.02 - 5.87 (m, 1H), 5.30 (qd, *J =* 1.8, 17.1 Hz, 1H), 5.17 (qd, *J =* 1.5, 10.5 Hz, 1H), 4.84 (dd, *J =* 12.0, 15.3 Hz, 2H), 4.42 - 4.37 (m, 1H), 4.33 - 4.27 (m, 1H), 4.10 - 4.05 (m, 2H), 3.72 (q, *J =* 10.5 Hz, 1H), 3.71 (q, *J =* 10.5 Hz, 1H), 2.48 (ddd, *J* = 2.1, 5.7, 13.5 Hz, 1H), 2.31 - 2.20 (m, 1H), 1.80 (d, *J* = 1.2 Hz, 3H) RMN - ¹³C 300 MHz, CDCl₃) δ (ppm) 164.0, 150.5, 136.0, 135.1, 134.0, 133.3, 133.2, 128.5, 128.0, 127.8, 126.6, 126.4, 126.2,125.7, 117.6, 111.0, 85.3, 84.1, 79.3, 72.4, 72.4, 71.6, 70.5, 38.1, 12.7

HRMS (ESI): Calculé pour C₂₄H₂₅O₅N₂ [M-H]⁻ 421,17635 ; trouvé 421,17520.

De la 2,6-lutidine (2éq., 575 µL, 4,53 mmol) est ajoutée à une solution du composé **12** (1 éq., 957 mg, 2,27 mmol) dans un mélange Dioxane/H₂O (3/1, 0,1 M, 22,7 mL). Une solution de tétroxyde d'osmium (2,5 w% dans du tert-butanol, 0,03 éq., 694 µL, 0,068 mmol) et le périodate de sodium (3 éq., 1,45 g, 6,80 mmol) sont ajoutés successivement au milieu réactionnel. Le milieu réactionnel blanc est agité à température ambiante pendant 4 à 5 h. La réaction est arrêtée avec de l'eau. Le milieu est extrait 3 fois avec du dichlorométhane. Les phases organiques sont rassemblées, lavées avec une solution saturée de Na₂S₂O₅, séchées sur Na₂SO₄, filtrées et concentrées sous pression réduite. Le brut réactionnel est purifié par chromatographie sur gel de silice (Acétone/Toluène 30/70 à 40/60 avec quelques gouttes de Et₃N) pour obtenir le composé **13** (634 mg, 66 %, mousse blanche).

Rf = 0,24 (EtOAc/Pentane 70/30)
IR (ATR) vₘₐₓ (cm⁻¹) 2931, 2858, 1706, 1647, 1462, 1249, 1150, 1109, 1049, 1000, 780, 624, 558, 417.

RMN - ¹H (300 MHz, CDCl₃) δ (ppm) 9.64 (s, 1 H), 9.65 (brs, 1H), 7.88 - 7.75 (m, 4 H), 7.57 (d, *J =* 1.2 Hz, 1H), 7.53 - 7.42 (m, 3H), 6.40 (dd, *J* = 5.7, 7.8 Hz, 1H), 4.73 (dd, *J =* 12.0, 30.6 Hz, 2H), 4.38 - 4.31 (m, 1H), 4.28 - 4.23 (m, 1H), 4.20 (s, 2H), 3.81 (dd, *J =* 2.7, 10.2 Hz, 1H), 3.66 (dd, *J =* 2.7, 10.2 Hz, 1H), 2.50 (ddd, *J* = 2.4, 6.0, 13.5 Hz, 1H), 2.23 - 2.11 (m, 1H), 1.89 (d, *J =* 1.2 Hz, 3H) RMN - ¹³C (75.5 MHz, CDCl₃) δ (ppm) 197.9, 163.8, 150.5, 136.0, 135.0, 133.4, 133.2, 129.2, 128.5, 128.4, 128.0, 127.9, 126.7, 126.5, 126.3, 125.7, 125.4, 111.3, 85.4, 83.6, 79.1, 71.8, 37.7, 12.6
HRMS (ESI): Calculé pour C₂₃H₂₃O₆N₂ [M-H]⁻ 423,15561 ; trouvé 423,15449. Du sel d'azolium bromure de *3*-éthyl-*5*-(2-hydroxyéthyl)-*4*-méthylthiazolium (0,3 éq., 14 mg, 0,056 mmol), préalablement séché sous vide pendant 1 jour à 45 °C est chargé dans un tube Schlenk et mis sous argon. Une solution du composé **13** (1,5 éq., 118 mg, 0,279 mmol) dans du THF (0,5 M, 557 µL) est ajoutée au tube Schlenk suivi par de la DBU distillée (0,3 éq., 8,3 µL, 0,056 mmol). Le milieu devient orange à la suite de la formation de l'intermédiaire de Breslow. Le dérivé d'α-tocophérol **9** (1,0 éq., 90 mg, 0,186 mmol) est ajouté au milieu qui est agité à 75 °C pendant 48 h. Après refroidissement à température ambiante, le milieu est concentré sous pression réduite. Le brut réactionnel est purifié par chromatographie sur gel de silice (Acétone/Toluène 20/80) pour obtenir le composé **14** (9 mg, 5 %, gomme incolore) conforme à l'invention.

Rf = 0,50 (Acétone/Toluène 20/80)
IR (ATR) vₘₐₓ (cm⁻¹) 2926.0, 2866.5, 1687.1, 1462.5, 1410.9, 1367.3, 1274.2, 1249.2, 1196.2, 1115.2, 1079.9, 959.4, 913.4, 856.4, 817.4, 754.1, 618.0, 561.1, 476.5, 417.7

RMN - ¹H (300 MHz, CDCl₃) δ (ppm) 8.19 (brs, 1H), 7.87 - 7.74 (m, 4H), 7.66 (s, 1H), 7.52 - 7.41 (m, 3H), 6.41 (dd, *J =* 5.4, 7.8 Hz, 1H), 4.71 (dd, *J =* 12.0, 27.6 Hz, 2H), 4.39 - 4.33 (m, 1H), 4.29 - 4.18 (m, 3H), 3.78 (dd, *J* = 2.7, 10.2 Hz, 1H), 3.62 (dd, *J =* , 10.2 Hz, 1H), 2.94 (t, *J =* 5.7 Hz, 2H), 2.70 (t, *J =* 6.3 Hz, 2H), 2.60 - 2.44 (m, 3H), 2.20 - 2.11 (m, 1H), 2.07 (s, 3H), 1.99 (s, 3H), 1.95 (s, 3H), 1.89 (s, 3H), 1.84 - 1.67 (m, 4H), 1.59 - 0.97 (m, 26H), 0.89 - 0.78 (m, 12H) RMN - ¹³C (75.5 MHz, CDCl₃) δ (ppm) 205.2, 171.6, 163.6, 150.3, 149.7, 140.5, 136.2, 135.1, 133.4, 133.2, 128.5, 128.0, 127.9, 126.7, 126.4, 126.2, 125.7, 125.0, 123.3, 117.6, 111.2, 85.3, 83.8, 79.3, 76.0, 75.2, 71.7, 71.7, 39.5, 37.8 - 37.4 (plusieurs C : CH, CH₂ aliphatiques), 33.3, 32.9, 32.8, 28.1, 27.4, 24.9, 24.6, 22.9, 22.8, 21.2, 20.7, 19.9, 19.8, 13.1, 12.6, 12.2, 12.0.

### Exemple 4

Un composé actif selon l'invention (Composé **16**) est préparé selon le schéma réactionnel montré sur la figure 4, à partir d'α-tocophérol **8** et du composé **4** dérivé de thymidine obtenu comme décrit dans l'Exemple 2.

De la 4-diméthylaminopyridine (DMAP) (quantité catalytique) et de l'anhydride succinique (1 éq., 75 mg, 0,752 mmol) sont ajoutés successivement à une solution du composé **4** (1 éq., 250 mg, 0,752 mmol) dans le toluène (0,044 M, 17,1 mL). Le milieu réactionnel est agité à reflux pendant 5 h. Après refroidissement à température ambiante, le milieu est concentré sous pression réduite pour obtenir le composé **15.**

De la DMAP (0,6 éq., 17 mg, 0,138 mmol), le composé diacide **15** (1,5 éq., 149 mg, 0,345 mmol) et du 1-éthyl-3-(3-diméthylaminopropyl)carbodiimide (EDC•HCl) (1,5 éq., 66 mg, 0,345 mmol) sont ajoutés successivement à une solution d'a-tocophérol **8** (1 éq., 99 mg, 0,230 mmol) dans du dichlorométhane (0,1 g/mL, 1 mL). Le milieu réactionnel est agité à température ambiante pendant 49 h. La réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 40/60) pour obtenir le composé **16** (232 mg, 52 % sur 2 étapes, mousse blanche) conforme à l'invention.
Rf = 0,10 (EtOAc/Pentane 20/80)
IR (ATR) vₘₐₓ (cm⁻¹) 3181.2, 2926.5, 2867.1, 1744.0, 1688.5, 1457.0, 1412.0, 1365.5, 1273.9, 1249.8, 1203.7, 1145.0, 1105.5, 1060.7, 963.8, 911.5, 736.4, 698.7, 606.2, 558.2, 486.2, 419.2
RMN - ¹H (300 MHz, CDCl₃) δ (ppm) 9.58 (brs, 1H), 7.40 - 7.27 (m, 6H), 6.32 - 6.24 (m, 1H), 4.57 - 4.39 (m, 3 H), 4.31 - 4.22 (m, 2 H), 4.15 - 4.08 (m, 1H), 3.03 - 2.92 (m, 2H), 2.81 - 2.72 (m, 2H), 2.63 - 2.55 (m, 2H), 2.49 (ddd, *J =* 3.0, 6.0, 13.8 Hz, 1H), 2.12 - 2.00 (m, 7H), 1.98 (s, 3H), 1.92 (s, 3H), 1.85 - 1.67 (m, 2H), 1.63 - 1.00 (m, 26 H), 0.93 - 0.78 (m, 12 H)
RMN - ¹³C (75.5 MHz, CDCl₃) δ (ppm) 172.0, 171.0, 164.0, 150.4, 149.6, 140.5, 137.3, 135.2, 128.6, 128.1, 127.8, 126.6, 124.9, 123.1, 117.5, 111.3, 83.4, 82.2, 78.3, 75.1, 71.8, 64.2, 39.4, 37.7 - 37.4 (plusieurs C : CH, CH₂ aliphatiques), 32.8, 32.7, 31.0, 29.0, 28.7, 28.0, 24.9, 24.5, 22.8, 22.7, 21.1, 20.6, 19.8, 19.8, 19.7, 13.0, 12.7, 12.2, 11.9.

### Exemple 5

Une composition pharmaceutique C1 conforme à l'invention répond à la composition indiquée dans le Tableau 1 ci-dessous.

**Tableau 1**

| Ingrédient | Quantité (mg) | % p/p |
|---|---|---|
| Composé **7** | 20 | 2 |
| α-tocophérol | 80 | 8 |
| Miglyol^{®} 812 | 100 | 10 |
| Lécithines E80 | 15 | 1,5 |
| Polysorbate 80 | 25 | 2,5 |
| Eau | Qsp 1000 | 100 |

Cette composition est obtenue comme suit :
- solubiliser le composé **7** conforme à l'invention et l'α-tocophérol libre dans l'huile Miglyol^{®} 812, pour former une phase lipophile,
- préparer une phase aqueuse comprenant au moins les lécithines E80 (agent tensioactif ionique) et le polysorbate 80 (agent tensioactif non ionique),
- chauffer séparément les 2 phases entre 70 et 80 °C,
- mélanger par inversion de phase en introduisant la phase aqueuse dans la phase lipophile à très haute vitesse dans un disperseur Ultra-Turrax^{®} de manière à former une émulsion grossière dont les globules lipidiques présentent un diamètre moyen supérieur à 2 µm,
- réduire la taille des globules lipidiques pour obtenir une nanoémulsion avec un homogénéiseur haute pression, un microfluidiseur ou une sonde à ultrason afin d'obtenir que 100 % des globules présentent une taille inférieure à 1 µm avec un diamètre moyen compris entre 140 et 300 nm,
- stériliser à l'autoclave à 121 °C pendant 15 min ou par filtration stérilisante. On obtient une nanoémulsion huile-dans-eau dans laquelle la prodrogue selon l'invention (composé **7**) se trouve dans la phase lipophile, sous une forme parfaitement solubilisée.

Le diamètre moyen (moyenne sur 3 mesures) mesuré par diffusion dynamique de la lumière (DLS) des gouttelettes de phase lipophile dispersées dans la phase aqueuse est de 235 nm, pour un indice de polydispersité PDI, mesuré par diffusion dynamique de la lumière (DLS), de 0,23. Le potentiel zeta, mesuré par diffusion électrophorétique de la lumière (ELS), de cette nanoémulsion conforme à l'invention est de -38,3 mV, compatible avec une répulsion électrostatique satisfaisante pour la stabilisation du système.

Cette composition est particulièrement utile, entre autres, pour le traitement des brûlures d'origine chimique, en particulier en application topique, et pour le traitement des maladies neurodégénératives, en particulier en administration par voie orale ou parentérale.

### Exemple 6

Une composition pharmaceutique C2 conforme à l'invention répond à la composition indiquée dans le Tableau 2 ci-dessous.

**Tableau 2**

| Ingrédient | Quantité (mg) | % p/p |
|---|---|---|
| Composé **16** | 20 | 2 |
| Miglyol^{®} 812 | 200 | 20 |
| Lécithines E80 | 12 | 1,2 |
| Polysorbate 80 | 25 | 2,5 |
| Eau | qsp 1000 | 100 |

Cette composition est obtenue en appliquant le protocole décrit dans l'Exemple 5.

On obtient une nanoémulsion huile-dans-eau dans laquelle la prodrogue selon l'invention (composé **16**) se trouve dans la phase lipophile, sous une forme totalement solubilisée.

Pour cette nanoémulsion conforme à l'invention, sont mesurés, le jour même (t = 0 jours), puis à plusieurs reprises au cours de 28 jours suivants (t = 1 jour, 2 jours, 3 jours, 7 jours, 14 jours, 21 jours, 28 jours) :
- le diamètre moyen des gouttelettes de phase lipophile dispersées dans la phase aqueuse, par diffusion dynamique de la lumière (DLS) ;
- l'indice de polydispersité PDI, par diffusion dynamique de la lumière (DLS) ;
- et le potentiel zeta de la nanoémulsion, par diffusion électrophorétique de la lumière (ELS).

Les résultats obtenus sont indiqués dans le Tableau 3 ci-dessous. Dans ce tableau, chaque valeur indiquée est la moyenne de trois mesures réalisées.

**Tableau 3**

| Temps (jours) | Diamètre gouttelettes de phase lipophile dispersées dans la phase aqueuse (nm) | PDI | Potentiel zeta (mV) |
|---|---|---|---|
| 0 | 157,9 +/- 0,6 | 0,101 +/- 0,012 | -48,9 +/- 1,4 |
| 1 | 159 +/- 2,1 | 0,113 +/- 0,013 | -40,9 +/- 0,4 |
| 2 | 159,3 +/- 1,6 | 0,087 +/- 0,014 | -40,9 +/- 0,3 |
| 3 | 157,8 +/- 2,3 | 0,11 +/- 0,013 | -26.0 +/- 1,6 |
| 7 | 159,1 +/- 1,7 | 0,11 +/- 0,021 | -19.4 +/- 1,1 |
| 14 | 159,8 +/- 3,4 | 0,105 +/- 0,024 | -37,5 +/- 1,3 |
| 21 | 160,6 +/- 1,7 | 0,1 +/- 0,001 | -38,9 +/- 1,1 |
| 28 | 161,5 +/- 1,7 | 0,098 +/- 0,012 | -33,7 +/- 1,2 |

On observe que pour chacun des 3 paramètres, les valeurs obtenues sont stables au cours du temps, ce qui témoigne d'une bonne stabilité de la nanoémulsion.

En moyenne, on obtient les résultats suivants :
- le diamètre moyen des gouttelettes de phase lipophile dispersées dans la phase aqueuse est de l'ordre de 160 nm ;
- avec un indice de polydispersité toujours inférieur à 0,2 ;
- et un potentiel zeta de l'ordre de -40 mV.

Cette composition est particulièrement utile, entre autres, pour le traitement des brûlures d'origine chimique, en particulier en application topique, et pour le traitement des maladies neurodégénératives, en particulier en administration par voie orale ou parentérale.

### Exemple 7 - Evaluation de la cytotoxicité d'une composition pharmaceutique sous forme de nanoémulsion conforme à l'invention sur des cellules neuronales humaines

Cette expérience est réalisée avec la composition C2 de l'Exemple 6, contenant le composé **16** en tant que composé actif.

Les lignées cellulaires suivantes sont utilisées :
- BE(2)-M17 (shScr-1, neuroblastomes dopaminergiques humains), obtenue auprès de l'ATCC^{®} (référence CRL-2267^{™}),
- sh403-1 (neuroblastome dopaminergique humain) décrite dans la publication de Dehay et al., PNAS, 2012, 109: 24, 9611-9616, présentant une inactivation (« knockdown ») stable de la fonction ATPase ATP13A2.

Ces lignées cellulaires sont cultivées en milieu OPTI-MEM (Life Technologies, 31985-047) en ajoutant 10% de sérum bovin fœtal (Sigma-Aldrich) et 1% de pénicilline / streptomycine. Pour toutes les expériences, des nanoémulsions fraîchement préparées sont utilisées et les cellules sont cultivées à 70-80% de confluence. Les cellules sont cultivées pendant 24 ou 48 h avec la nanoémulsion diluée au 1000^{ème} dans le milieu de culture. Un témoin non traité est également réalisé. Chaque expérience est reproduite au moins trois fois. La viabilité cellulaire est estimée par le test MTS (ATCC / LGC Promochem) en suivant les instructions du fabricant.

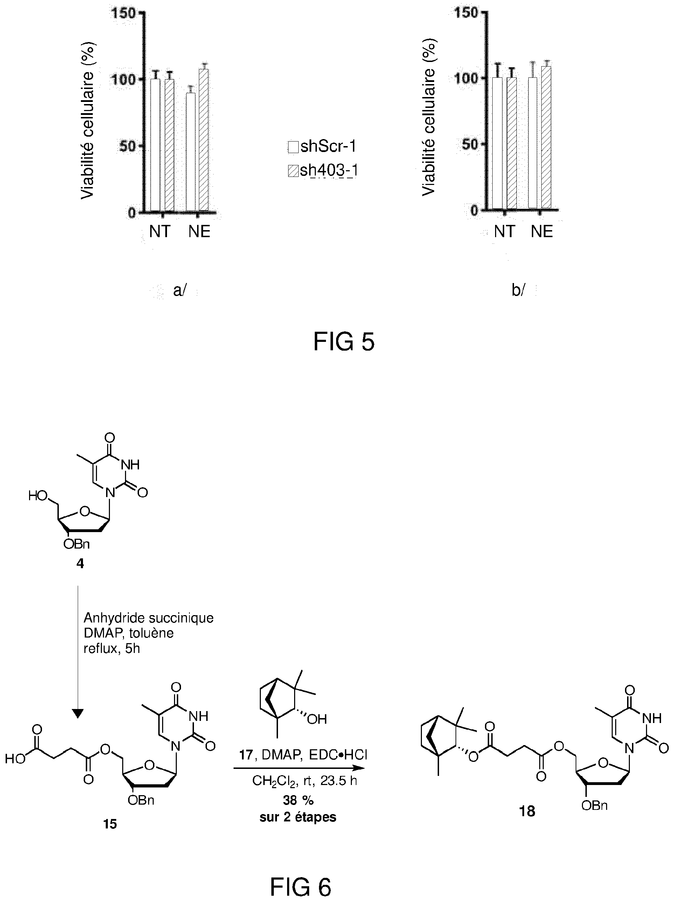

Les résultats obtenus sont montrés sur la figure 5. On observe que la viabilité cellulaire des cellules traitées par la nanoémulsion conforme à l'invention (« NE ») est sensiblement égale à celle des cellules non traitées (« NT »), démontrant l'absence de toxicité du composé actif **16** selon l'invention.

### Exemple 8

Un composé actif selon l'invention (Composé **18**) est préparé selon le schéma réactionnel montré sur la figure 6, à partir de fenchol **17** et du dérivé de thymidine **15** obtenu comme décrit dans l'exemple 4 à partir du composé **4.**

Le composé acide **15** (431 mg, 1,00 mmol, 1,5 éq.), de la DMAP (49 mg, 0,40 mmol, 0,6 éq.) et du 1-éthyl-3-(3-diméthylaminopropyl)carbodiimide (EDC•HCl) (1,5 éq., 191 mg, 1,0 mmol sont ajoutés successivement à une solution de fenchol **17** (1 éq., 103 mg, 0,66 mmol) dans du dichlorométhane (0,1 g/mL, 1 mL). Le milieu réactionnel est agité à température ambiante pendant 23,5 h. La réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (EtOAc/Pentane 50/50) pour obtenir le composé **18** (195 mg, 38 % sur 2 étapes à partir de **4,** gomme translucide).
Rf = 0,36 (EtOAc/P 50/50) ;
IR (ATR) υₘₐₓ (cm⁻¹) 3674, 2961, 1685, 1456, 1408, 1379, 1273, 1201, 1158, 1046, 1006, 909, 731, 697, 647, 604, 558, 488, 420 ;
RMN ¹H (300 MHz, CDCl3) δ (ppm) 10.04 (s, 1H, NH), 7.40-7.16 (m, 6H), 6.26 (dd, *J* = 6.9 Hz, 1H), 4.50 (système AB, *J* = 11.7 Hz, 2H), 4.40-4.28 (m, 2H), 4.28-4.16 (m, 2H), 4.14-4.04 (m, 1H), 2.75-2.55 (m, 4H), 2.47 (ddd, *J =* 3.0, 6.3, 13.8 Hz, 1H), 2.13-1.98 (m, 1H), 1.89 (s apparaissant, 3H), 1.75-1.56 (m, 3H), 1.56-1.47 (m, 1H), 1.46-1.19 (m, 2H), 1.18-1.10 (m, 1H), 1.04 (s, 3H), 0.99 (s, 3H), 0.72 (s, 3H) ;
RMN ¹³C (75.5 MHz, CDCl3) δ (ppm) 172.4, 171.9, 164.1, 150.5, 137.2, 135.2, 128.5, 127.9, 127.6, 111.1, 86.7, 85.2, 82.1, 78.2, 71.6, 64.1, 48.2, 48.1, 41.2, 39.3, 37.4, 29.6, 29.0, 28.9, 26.5, 25.7, 20.0, 19.3, 12.6.

### Exemple 9 - exemple comparatif dépourvu de groupement lipidant

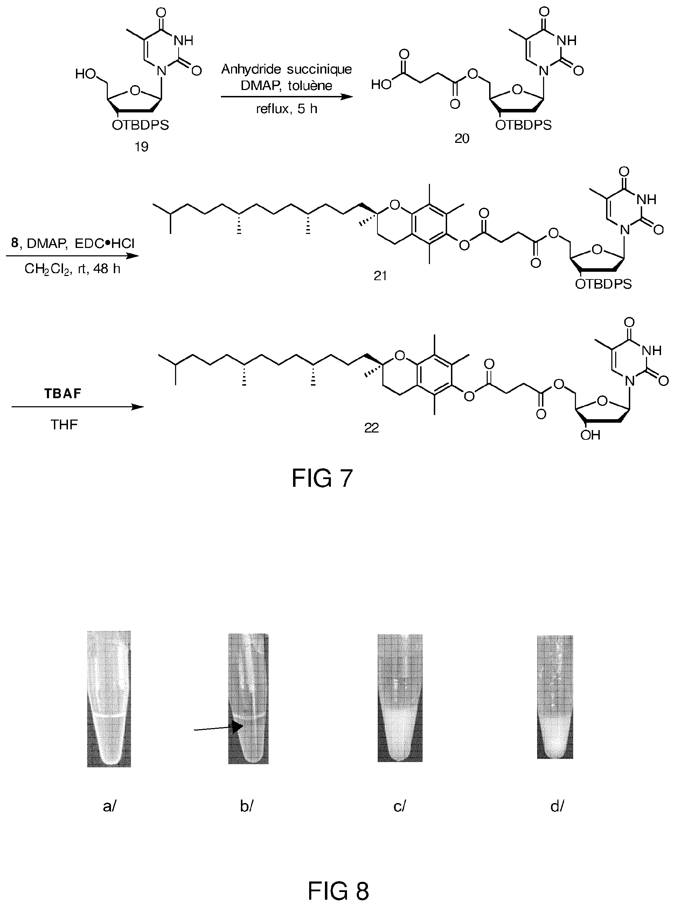

Un composé non conforme à l'invention (Composé **22**), ne comportant pas de groupement lipidant conforme à l'invention, est préparé selon le schéma réactionnel montré sur la figure 7, à partir d'α-tocophérol **8** et d'un dérivé de thymidine, 3'-O-tertbutyldiphénylsilylthymidine **19** obtenu comme décrit dans la publication de Cunha et al., ACS Omega, 2020, 5, 11, 5815-5823.

### Synthèse du composé 19

Le composé 19 a préalablement été obtenu comme suit :
1/ à une solution de thymidine (1 équiv, 6 g, 24,77 mmol) dans la pyridine (118 mL) et sous argon ont été ajoutés séquentiellement du chlorure de tert-butyldiméthylsilyle (TBDMSCI) (1,2 équiv, 4,48 g, 29,72 mmol) et de la 4-diméthylaminopyridine (DMAP) (pointe de spatule). Le mélange a été agité pendant une nuit à température ambiante jusqu'à la fin de la réaction. La réaction a ensuite été stoppée par l'ajout de NaHCOs (10 ml, aq.) et diluée avec de l'eau (10 ml). La phase aqueuse a été extraite trois fois avec du dichlorométhane (DCM) (3 x 30 mL), et les phases organiques réunies ont été séchées sur Na₂SO₄ avant concentration à sec sous vide. Le brut obtenu a été purifié par chromatographie flash sur gel de silice (pentane/EtOAc, 70/30) pour obtenir un composé sous forme de poudre blanche (7,68 g, 21,55 mmol, 87%).
2/ à une solution de ce composé (1 équiv, 14,7 g, 41,21 mmol) dans le diméthylformamide (DMF) (88 mL) et sous argon ont été ajoutés séquentiellement de l'imidazole (1,2 équiv, 3,37 g, 49,54 mmol) du tert-butylchlorodiphénylsilane (TBDPSCI) (1,2 équiv , 12,88 mL, 49,54 mmol). Le mélange a été agité pendant une nuit à température ambiante jusqu'à la fin de la réaction. Le mélange a ensuite été dilué avec du toluène (100 mL), et le DMF évaporé sous vide pour obtenir une huile jaune. L'huile a été diluée dans de l'éther diéthylique (10 mL), et le précipité blanc formé a été filtré et lavé avec une solution aqueuse de NaCl. La phase organique a été séchée sur Na₂SO₄ avant concentration à sec sous vide. Un composé a été obtenu sous la forme d'une huile jaune.
3/ à une solution de ce composé dans le méthanol (80 mL) et sous argon a été ajouté de l'acide para-toluènesulfonique monohydraté (0,471 g, 2,477 mmol). Le mélange a été agité pendant une nuit à température ambiante jusqu'à la fin de la réaction. Le méthanol a ensuite été évaporé sous vide pour obtenir une huile jaune. Le produit brut a été dilué avec de l'EtOAc (30 ml) et la phase organique lavée trois fois avec du NaHCO₃ (10 ml, aq.) et de la saumure (3 x 20 ml). La phase organique a été séchée sur Na₂SO₄ avant concentration à sec sous vide. Le brut résultant a été purifié par chromatographie flash sur gel de silice (éther de pétrole/EtOAc, 50/50) pour obtenir le composé **19** attendu sous forme d'une poudre blanche (6,90 g, 14,36 mmol, 58 % pour les étapes 2/ + 3/).

Rf = 0,29 (éther de pétrole/EtOAc, 50/50) ;
RMN ¹H (300 MHz, CDCl₃) δ ppm : 9,18 (bs, 1H, NH), 7,59-7,69 (m, 4H, H Ar), 7,34-7,50 (m, 6H, H Ar), 7,30 (d, J = 1,2 Hz 1H, H6), 6,26 (dd, J = 6,0 Hz, 7,8 Hz, 1H, H1'), 4,41-4,48 (m, 1H, H3'), 3,94-4,00 (m, 1H, H4'), 3,62 (dd, J = 2,4 Hz, 12,0 Hz, 1H, H5b'), 3,24 (dd, J = 3,0 Hz, 12,3 Hz, 1H, H5a'), 2,43 (bs, 1H, OH), 2,26 (ddd, J = 3,0 Hz, 6,0 Hz, 13,2 Hz, 1H, H2b'), 2,04-2,22 (m, 1H, H2a'), 1,82 (d, J = 1,2 Hz, 3H, H7), 1,08 (s, 9H, tBu) ;
RMN ¹³C (75,46 MHz, CDCl₃) δ ppm : 164,0 (C4), 150,5 (C2), 136,9 (C6), 135,8 (CH Ar), 133,4 (Cq Ar), 133,3 (Cq Ar), 130,2 (CH Ar), 130,2 (CH Ar), 128,0 (CH Ar), 111,0 (C5), 87,8 (C4'), 86,6 (C1'), 73,1 (C3'), 62,1 (C5'), 40,4 (C2'), 27,0 (CH3tBu ), 19,1 (Cq tBu), 12,5 (C7) ;
IR (ATR) v (cm⁻¹) : 2932, 1682, 1471, 1274, 1104, 1031, 740, 702.

### Synthèse du composé 22

La DMAP (quantité catalytique) et l'anhydride succinique (1 éq., 42,4 mg, 0,424 mmol) sont ajoutés successivement à une solution du composé **19** (1 éq., 204 mg, 0,424 mmol) dans le toluène (0,1 M, 4,24 mL). Le milieu réactionnel est agité à reflux pendant 6 h. Après refroidissement à température ambiante, le milieu est concentré sous pression réduite pour obtenir le composé **20** qui est engagé dans la réaction suivante sans purification ultérieure.

La DMAP (0,6 éq., 20,7 mg, 0,169 mmol), le composé **20** (1,5 éq., 0,424 mmol) et l'EDC•HCl (1,5 éq., 81 mg, 0,424 mmol) sont ajoutés successivement à une solution d'a-tocophérol **8** (1 éq., 121,7 mg, 0,282 mmol) dans du dichlorométhane (0,1 g/mL, 1 mL). Le milieu réactionnel est agité à température ambiante pendant 48 h. La réaction est arrêtée avec une solution saturée de NH₄Cl. Le milieu est extrait 3 fois avec du dichlorométhane. Les phases organiques sont rassemblées, séchées sur Na₂SO₄, filtrées et concentrées sous pression réduite. Le brut réactionnel contenant le composé **21** est utilisé sans purification ultérieure dans la réaction suivante.

Du fluorure de tétrabutylammonium TBAF (c=1M dans THF, 1 éq., 282 µl, 0,282 mmol), est ajouté sur une solution du composé **21** (0,282 mmol) à 0°C. Lorsque la température a atteint la température ambiante, la réaction est agitée 2 h. La réaction est arrêtée avec H₂O. Le milieu est extrait 3 fois avec du dichlorométhane. Les phases organiques sont rassemblées, séchées sur Na₂SO₄, filtrées et concentrées sous pression réduite. Le brut réactionnel est purifié par chromatographie sur gel de silice (Toluène/acétone 70/30) pour obtenir le composé **22** (177 mg, 45 % sur 3 étapes solide blanc).
Rf = 0,25 (toluène/acétone 70/30) ;
IR (ATR) vₘₐₓ (cm⁻¹) 3456, 3178, 2926, 2867, 1742, 1686, 1461, 1412, 1373, 1242, 1145, 1100, 914, 736, 695, 606, 489, 417 ;
RMN ¹H (300 MHz, CDCl₃) δ (ppm) 9.76 (brs, 1H), 7.30 (s, 1H), 6.28 (t, 1H), 4.43 (dd, *J* = 3.0, 12 Hz, 1H), 4.34 (brs, 1 H), 4.27 (dd, *J* = 3.0, 12 Hz, 1H), 4.17 - 4.07 (m, 1H), 3.63 (brs, 1H), 3.03 - 2.91 (m, 2H), 2.84 - 2.71 (m, 2H), 2.63 - 2.51 (m, 2H), 2.47 - 2.34 (m, 1H), 2.19 - 2.04 (m, 1H), 2.07 (s, 3H), 2.0 (s, 3H), 1.96 (s, 3H), 1.89 (s, 3H), 1.85 - 1.65 (m, 2H), 1.63 - 0.97 (m, 26 H), 0.93 - 0.76 (m, 12 H) ;
RMN ¹³C (75.5 MHz, CDCl₃) δ (ppm) 172.2, 171.2, 164.2, 150.8, 149.6, 140.5, 137.5, 126.7, 124.9, 123.1, 117.5, 111.4, 85.2, 84.3, 75.2, 71.4, 64.3, 40.2, 39.4, 37.6, 37.5, 37.5, 37.3, 32.8, 32.8, 29.1, 28.7, 28.0, 24.9, 24.5, 22.8, 22.7, 21.1, 20.6, 19.8-19.7 (plusieurs C : CH, CH₂ aliphatiques), 13.0, 12.6, 12.2, 11.9.

### Exemple 10 - Solubilité dans l'huile

Le composé conforme à l'invention **16** et le composé comparatif **22,** dépourvu de groupement lipidant, sont soumis à un essai de solubilité dans le Miglyol^{®} 812, à différentes concentrations entre 1 et 5 % p/p.

Les résultats sont montrés sur la figure 8. Comme on peut l'observer, en a/ sur cette figure, à la concentration de 5 % p/p, le composé conforme à l'invention **16** est totalement soluble dans le véhicule huileux. Le composé comparatif **22** s'avère quant à lui insoluble : dès la concentration de 1 % p/p, en b/ sur la figure, on observe la présence d'une interface dans le milieu, indiquée par une flèche, témoignant d'une non miscibilité des phases en présence. Ce phénomène est plus net encore aux concentrations de 2 % p/p et de 5 % p/p montrées respectivement en c/ et en d/ sur la figure. Ce résultat démontre l'impossibilité de formuler le composé comparatif **22** dans une composition sous la forme particulièrement avantageuse de nanoémulsion, contrairement au composé conforme à l'invention **16.**

### Exemple 11 - Irradiation UV

Cet essai a été réalisé sur le composé conforme à l'invention **16,** au moyen d'une ampoule simulant la lumière du soleil Osram Ultra Vitalux 300 W 230 V, à rayonnement UVA dans la plage de 315 à 400 nm (13,6 W) et UVB dans la plage de 280 à 315 nm (3,0 W).

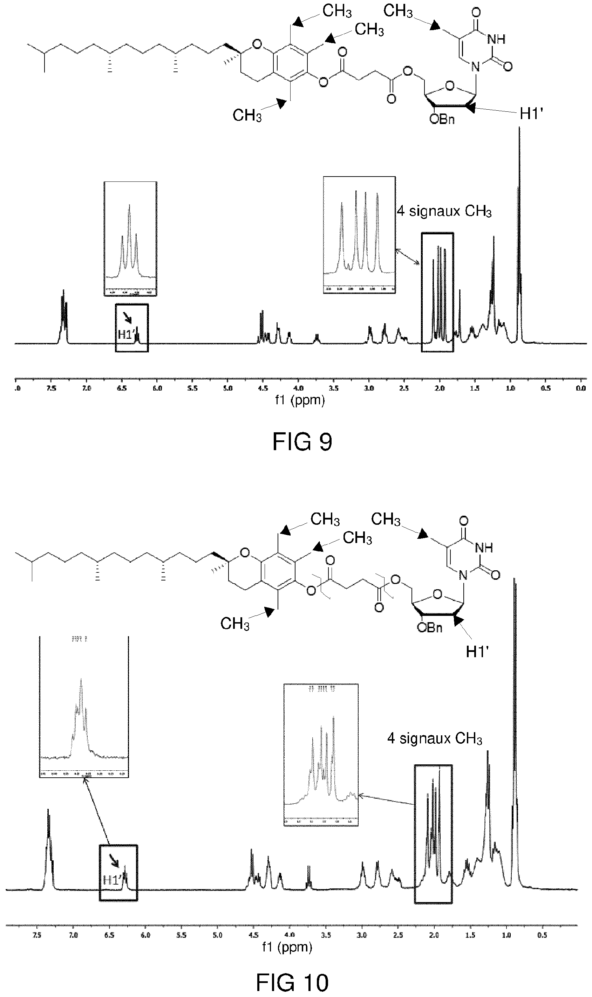

Le composé a été placé dans le solvant deutérié CDCl₃ (30mg dans 750 µl) pour suivre la réaction par RMN ¹H. Avant irradiation, les signaux des groupements méthyle (CH₃) du squelette tocophérol et de la nucléobase et le signal anomérique H1' du nucléoside ont une multiplicité normale, comme montré sur la figure 9 : 4 singulets pour les méthyles et un triplet pour l'anomérique. Au bout de 8 h d'irradiation, comme montré sur la figure 10, un dédoublement des signaux de ces groupements méthyle et du signal anomérique du nucléoside fait apparaitre des signaux superposés attestant la présence de deux autres composés, issus du clivage, induit par le rayonnement ultra-violet, des fonctions ester, comme indiqué par des traits en zigzag sur la figure.

Ces résultats laissent présager d'un clivage plus rapide encore du composé, pour libérer l'α-tocophérol, au sein d'une nanoémulsion, c'est-à-dire en présence d'eau, bon nucléophile favorisant la coupure de la liaison ester.

### Exemple 12 - Etude de l'effet antioxydant sur kératinocytes

Cette étude a été réalisée sur le composé conforme à l'invention **16** décrit ci-avant.

Deux compositions pharmaceutiques C3 et C4 conformes à l'invention répondant aux compositions indiquées dans le Tableau 4 ci-dessous, ont été préparées.

**Tableau 4**

| Ingrédient | C3 (% p/p) | C4 (% p/p) |
|---|---|---|
| Composé **16** | 3,0 | 5,0 |
| Lécithines E80 | 1,2 | 1,2 |
| Miglyol^{®} 812 | 15,8 | 13,8 |
| Tween^{®} 80 | 2,5 | 2,5 |
| Glycérol | 2,3 | 2,3 |
| Eau Milli-Q^{®} ou p.p.i. | qsp 100 | qsp 100 |

Le protocole suivant a été mis en œuvre à cet effet.

Une phase huileuse a été préparée en dispersant les lécithines E80 et le composé **16** dans le Miglyol^{®} 812 sous ultrasons à 70°C, jusqu'à dispersion complète. Une phase aqueuse a été préparée par dispersion du Tween^{®} 80 et du glycérol dans de l'eau Milli-Q^{®} ou pour préparation injectable (p.p.i.). Une inversion de phase a été réalisée avec 800 mg de la phase aqueuse et 200 mg de la phase huileuse. Le mélange a été vortexé puis soniqué à la sonde pendant 8 min.

L'évaluation de l'effet antioxydant des compositions C3 et C4 selon l'invention a été basée sur le dosage de la quantité d'espèces réactives de l'oxygène (ROS) produites dans le milieu par cytométrie en flux, après mise en présence de cellules traitées par ces composition avec l'indicateur de stress oxydatif CM-H₂DCFDA (5-(et-6)-chlorométhyl-2',7'-dichlorodihydrofluorescéine diacétate, ester d'acétyle) (Invitrogen^{®}). Par clivage de ses groupes acétates par des estérases et son oxydation intracellulaire par les ROS, cet indicateur non fluorescent est converti en dichlorofluorescéine 2',7' (DCF) hautement fluorescente. La mesure de l'intensité de fluorescence permet ainsi de déduire la quantité de DCF, et par voie de conséquence de ROS, dans le milieu cellulaire.

Des kératinocytes primaires isolés de plastie mammaire et immortalisés par introduction et surexpression du gène de la transcriptase inverse de la télomérase hTERT et introduction de l'antigène T du virus SV40, ont été utilisés pour ces expériences. Le milieu de culture est le milieu KGM2 (Keratinocyte Growth Medium 2) (Promocell^{®}), et la température de culture 37 °C.

Dans une 1^{ère} expérience, des cellules ont été incubées avec la composition C4 diluée au 1/1000^{ème} dans le milieu de culture pendant 30 min. Elles ont ensuite été rincées avec du tampon phosphate salin (PBS), puis irradiées avec un simulateur solaire (Suntest CPS+, Atlas) (300-800 nm), afin d'induire la production de ROS, pendant 2 min à 765 W/m² (90 K) dans du PBS additionné de composition C4 diluée à 1/1000^{ème}. Elles ont enfin été incubées à nouveau avec la composition C4 diluée au 1/1000^{ème} dans le milieu de culture pendant 30 min. Un contrôle négatif a été réalisé de la même manière, sans composition C4.

Les cellules ont ensuite été décollées par tryptinisation au moyen de trypsine à 0,0025 % et EDTA à 0,01 % pendant 10 min à 37 °C, neutralisées par sérum de veau fœtal à 10 % et séparées du milieu par centrifugation. Elles ont été incubées pendant 20 min à 37 °C avec la sonde CM-H₂DCFDA à une concentration finale de 1 M (volume d'incubation 100 µl). La fluorescence a ensuite été lue par un cytomètre en flux Accuri^{®} 6.

Les résultats obtenus, exprimés en % d'intensité de fluorescence moyenne (MFI) par rapport à la condition contrôle non irradiée (et sans C4), sont montrés sur la figure 11. On observe que la composition C4 selon l'invention inhibe la production de ROS (baisse de l'intensité de la fluorescence, témoignant d'une baisse de production de DCF à partir de CM-H₂DCFDA), alors que sans cette composition l'irradiation induit une production de ROS de l'ordre de 20 %.

Dans une 2^{ème} expérience, des cellules ont été incubées avec la composition C4 diluée au 1/1000^{ème} dans le milieu de culture, la composition C3 diluée au 1/1000^{ème} dans le milieu de culture, ou l'α-tocophérol à 0,5 % p/v dans le milieu de culture, pendant 48 h. Les cellules ont ensuite été traitées comme décrit ci-dessus, avec la composition C4 diluée au 1/1000^{ème} dans le milieu de culture, la composition C3 diluée au 1/1000^{ème} dans le milieu de culture, ou l'α-tocophérol à 0,5 % p/v dans le milieu de culture, pour l'irradiation et l'incubation post-irradiation.

Les résultats obtenus, exprimés en % d'intensité de fluorescence moyenne (MFI) par rapport à la condition contrôle sans additif et non irradiée, sont montrés sur la figure 12. L'α-tocophérol à 0,5 % p/v, contrôle positif antioxydant à cette concentration, inhibe fortement la production de ROS induite par le rayonnement UV ainsi que les ROS intracellulaires. La composition selon l'invention C3 inhibe partiellement la production de ROS induite par le rayonnement ultraviolet. La composition selon l'invention C4, plus concentrée en composé actif, inhibe fortement la production de ROS UV-induits, ainsi que les ROS cytoplasmiques.

L'ensemble de ces résultats démontre une bonne efficacité antioxydante de la composition conforme à l'invention C4, ainsi que, dans une moindre mesure, de la composition conforme à l'invention C3.

## Revendications

1. Prodrogue **caractérisée en ce qu'**elle consiste en :
- un composé dit actif comprenant :
une molécule antioxydante pharmaceutiquement active couplée de manière covalente, par une liaison comprenant au moins un groupe ester carboxylique, à un nucléoside comprenant un motif ribose ou désoxyribose lié de manière covalente à une base nucléique,
ledit nucléoside portant au moins un groupement, dit groupement lipidant, choisi parmi les groupements -R et -CO-R, dans lesquels R représente un radical hydrocarboné, linéaire, ramifié et/ou cyclique, saturé et/ou insaturé, aromatique ou non, éventuellement substitué, éventuellement interrompu par un ou plusieurs hétéroatomes et/ou par un ou plusieurs groupements comprenant au moins un hétéroatome ou au moins groupement comprenant au moins un hétéroatome, R ne comprenant aucun groupement hydroxyle, amine primaire, amine secondaire, phosphate ou thiol, et pouvant comporter un cycle ou plusieurs cycles, optionnellement condensés,
chacun des groupements hydroxyles dudit motif ribose ou désoxyribose non impliqué dans une liaison à ladite molécule antioxydante ou à un groupement lipidant étant substitué par un groupement protecteur d'une fonction hydroxyle fixé sur l'atome d'oxygène dudit groupement hydroxyle et choisi parmi les radicaux hydrocarbonés linéaires, ramifiés et/ou cycliques, saturés et/ou insaturés, aromatiques ou non, éventuellement substitués, éventuellement interrompus par un ou plusieurs hétéroatomes et/ou par un ou plusieurs groupements comprenant au moins un hétéroatome ou au moins un groupement comprenant au moins un hétéroatome, à l'exclusion des groupements hydroxyle, amine primaire, amine secondaire, phosphate et thiol, et pouvant comporter un cycle ou plusieurs cycles, le cas échéant un ou plusieurs hétérocycles comprenant un ou plusieurs hétéroatomes, lesdits cycles étant optionnellement condensés,
et chacun des groupements amines primaires non impliqué dans une liaison à ladite molécule antioxydante ou à un groupement lipidant étant substitué par un groupement protecteur d'une fonction amine primaire fixé sur l'atome d'azote dudit groupement amine primaire et choisi parmi les radicaux hydrocarbonés linéaires, ramifiés et/ou cycliques, saturés et/ou insaturés, aromatiques ou non, éventuellement substitués, éventuellement interrompus par un ou plusieurs hétéroatomes et/ou par un ou plusieurs groupements comprenant au moins un hétéroatome ou au moins un groupement comprenant au moins un hétéroatome, à l'exclusion des groupements hydroxyle, amine primaire, amine secondaire, phosphate et thiol, et pouvant comporter un cycle ou plusieurs cycles, le cas échéant un ou plusieurs hétérocycles comprenant un ou plusieurs hétéroatomes, lesdits cycles étant optionnellement condensés,
- ou un des sels pharmaceutiquement acceptables dudit composé actif.

2. Prodrogue selon la revendication 1, dans laquelle ledit groupement lipidant est choisi parmi :
- les groupements -CH₂-R', -CO-CH₂-R' et -CO-(CH₂)₂-CO-O-R', où R' représente le cholestérol, le tocophérol, le tocotriénol, un noyau phényle éventuellement substitué par un ou plusieurs groupements R₅, un noyau naphtyle éventuellement substitué par un ou plusieurs groupements R₅, ou un noyau pyridine éventuellement substitué par un ou plusieurs groupements R₅, où R₅ représente une chaine hydrocarbonée linéaire ou ramifiée, saturée ou insaturée en C1-C25,
- un radical allyle ou un radical propargyle,
- les groupements -R et -CO-R, où R représente : une chaine hydrocarbonée linéaire ou ramifiée, saturée ou insaturée en C1-C25 ; un groupement -(CH₂)₂-O-R₅-O-CH₃ où R₅ représente une chaine hydrocarbonée linéaire ou ramifiée, saturée ou insaturée en C1-C25 ; un groupement -CH₂-CH(O-R₆)-O-R₇ où R₆ et R₇, identiques ou différents, représentent chacun une chaine hydrocarbonée linéaire ou ramifiée, saturée ou insaturée en C1-C25 ; un groupement -CH(OR₆)-O-R₇ où R₆ et R₇, identiques ou différents, représentent chacun une chaine hydrocarbonée linéaire ou ramifiée, saturée ou insaturée en C1-C25 ; ou un groupement -CH₂-CH(O-CO-R₆)-O-CO-R₇ où R₆ et R₇, identiques ou différents, représentent chacun une chaine hydrocarbonée linéaire ou ramifiée, saturée ou insaturée en C1-C25,
- les groupements -CH₂-R₈ où R₈ représente un groupement triazole, un groupement benzothiophène, un groupement benzofurane ou un groupement indole, R₈ étant éventuellement substitué par un ou plusieurs groupements R₅ où R₅ représente une chaine hydrocarbonée linéaire ou ramifiée, saturée ou insaturée en C1-C25,
- les groupements -(CH₂)₂-O-(CH₂)₂-O-(CH₂)ₚ-O-CH₂-R₁₅, où p est un nombre entier compris entre 2 et 25 et R₁₅ représente un atome d'hydrogène ou un groupement phényle ;
- les groupements -CO-CH₂-O-(CH₂)_{q}-O-CH₂-R₁₆, où q est un nombre entier compris entre 2 et 25 et R₁₆ représente un atome d'hydrogène ou un groupement phényle ;
- un groupement triazole, le cas échéant substitué par une chaine hydrocarbonée linéaire ou ramifiée, saturée ou insaturée en C1-C25.

3. Prodrogue selon l'une des revendications 1 ou 2, dans laquelle le couplage de ladite molécule antioxydante audit nucléoside est réalisé sur un premier atome d'oxygène dudit nucléoside choisi parmi l'atome d'oxygène situé en position 5' dudit motif ribose ou désoxyribose et l'atome d'oxygène situé en position 3' dudit motif ribose ou désoxyribose.

4. Prodrogue selon la revendication 3, dans laquelle ledit groupement lipidant est porté par un deuxième atome d'oxygène dudit nucléoside, différent dudit premier atome d'oxygène, choisi parmi l'atome d'oxygène situé en position 5' dudit motif ribose ou désoxyribose et l'atome d'oxygène situé en position 3' dudit motif ribose ou désoxyribose, et/ou par un atome d'azote d'un groupement amine de ladite base nucléique.

5. Prodrogue selon l'une quelconque des revendications 1 à 4, dans laquelle ledit nucléoside présente la formule générale (I) : 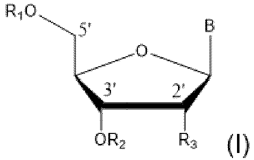 dans laquelle
B représente ladite base nucléique, éventuellement substituée au niveau d'un groupement amine par un groupement lipidant, chacun des groupements amines primaires éventuels de ladite base nucléique non impliqué dans une liaison à ladite molécule antioxydante ou à un groupement lipidant étant substitué par un groupement protecteur d'une fonction amine primaire tel que défini dans la revendication 1,
et
- soit R₁ représente la liaison à ladite molécule antioxydante, et
R₂ représente un groupement lipidant,
R₃ représente H ou OR₄ où R₄ représente un groupement lipidant ou un groupement protecteur d'une fonction hydroxyle tel que défini dans la revendication 1,
ou R₂ et R₄ forment ensemble, avec les atomes d'oxygène auxquels ils sont rattachés, un groupement acétal comprenant une ou plusieurs chaines hydrocarbonée(s) linéaire(s), ramifiée(s) et/ou cycliques, saturée(s) ou insaturée(s), aromatiques ou non, éventuellement substituées, éventuellement interrompues par un ou plusieurs hétéroatomes et/ou par un ou plusieurs groupements comprenant au moins un hétéroatome ou au moins un groupement comprenant au moins un hétéroatome, à l'exclusion des groupements hydroxyle, amine primaire, amine secondaire, phosphate et thiol, et pouvant comporter un cycle ou plusieurs cycles, le cas échéant un ou plusieurs hétérocycles comprenant un ou plusieurs hétéroatomes, les cycles étant optionnellement condensés,
- soit R₁ représente la liaison à ladite molécule antioxydante, et
R₂ représente un groupement lipidant ou un groupement protecteur d'une fonction hydroxyle tel que défini dans la revendication 1,
R₃ représente OR₄ où R₄ représente un grupement lipidant,
- soit R₂ représente la liaison à ladite molécule antioxydante, et
R₁ représente un groupement lipidant,
R₃ représente H ou OR₄ où R₄ représente un groupement lipidant ou un groupement protecteur d'une fonction hydroxyle tel que défini dans la revendication 1,
- soit R₂ représente la liaison à ladite molécule antioxydante, et
R₁ représente un groupement lipidant ou un groupement protecteur d'une fonction hydroxyle tel que défini dans la revendication 1,
R₃ représente OR₄ où R₄ représente un groupement lipidant.

6. Prodrogue selon l'une quelconque des revendications 1 à 5, dans laquelle ladite molécule antioxydante est couplée de manière covalente audit nucléoside par l'intermédiaire d'un bras espaceur contenant au moins un groupe ester carboxylique et/ou au moins un groupement carbonyle lié à l'atome d'oxygène en position 5' dudit motif ribose ou désoxyribose ou à l'atome d'oxygène en position 3' dudit motif ribose ou désoxyribose ou à un atome d'oxygène de ladite molécule antioxydante.

7. Prodrogue selon l'une quelconque des revendications 1 à 6, dans laquelle ladite molécule antioxydante est couplée de manière covalente audit nucléoside par l'intermédiaire d'un bras espaceur de formule générale (II) : 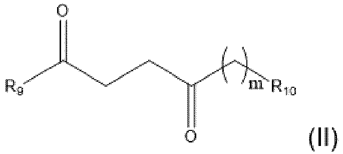 dans laquelle R₉ représente la molécule antioxydante et R₁₀ représente le nucléoside, et
- m est égal à 0 et la liaison de la molécule antioxydante au bras espaceur est portée par un atome d'oxygène de la molécule antioxydante et/ou la liaison du nucléoside au bras espaceur est portée par un atome d'oxygène du nucléoside,
- ou m est égal à 1 et la liaison de la molécule antioxydante au bras espaceur est portée par un atome d'oxygène de la molécule antioxydante.

8. Prodrogue selon l'une quelconque des revendications 1 à 7, dans laquelle ladite molécule antioxydante est choisie parmi l'α-tocophérol, le β-tocophérol, le γ-tocophérol, l'α-tocotriénol, le β-tocotriénol, le γ-tocotriénol, le δ-tocotriénol, le resvératrol, l'acide lipoïque, le rétinol, le rétinal, l'acide rétinoïque et l'acide ascorbique.

9. Composition pharmaceutique **caractérisée en ce qu'**elle contient une prodrogue selon l'une quelconque des revendications 1 à 8 dans un véhicule pharmaceutiquement acceptable.

10. Composition pharmaceutique selon la revendication 9, sous forme d'une émulsion comprenant une phase lipophile et une phase aqueuse, ladite prodrogue étant contenue dans ladite phase lipophile, de préférence sous forme de nanoémulsion.

11. Prodrogue selon l'une quelconque des revendications 1 à 8 ou composition pharmaceutique selon l'une quelconque des revendications 9 à 10 pour son utilisation en tant que médicament.

12. Prodrogue ou composition pharmaceutique pour son utilisation selon la revendication 11, pour le traitement des brûlures chimiques ou radiologiques.

13. Prodrogue ou composition pharmaceutique pour son utilisation selon la revendication 12, selon laquelle ladite prodrogue ou composition pharmaceutique est administrée par voie topique.

14. Prodrogue ou composition pharmaceutique pour son utilisation selon la revendication 11, pour le traitement d'une maladie neurodégénérative.

15. Prodrogue ou composition pharmaceutique pour son utilisation selon la revendication 14, selon laquelle ladite prodrogue ou composition pharmaceutique est administrée par voie orale ou parentérale.

## Patentansprüche

1. Prodrug, **dadurch gekennzeichnet, dass** es aus Folgendem besteht:
- eine aktive Verbindung, umfassend:
ein pharmazeutisch aktives Antioxidationsmolekül, das über eine Bindung, die mindestens eine Carbonsäureestergruppe umfasst, kovalent mit einem Nukleosid gekoppelt ist, das ein Ribose- oder Deoxyribose-Muster umfasst, das kovalent mit einer Nukleobase verbunden ist,
wobei das Nukleosid mindestens eine Gruppe, eine sogenannte Lipidisierungsgruppe, trägt, ausgewählt aus den Gruppen -R und -CO-R, wobei R einen linearen, verzweigten und/oder cyclischen, gesättigten und/oder ungesättigten, aromatischen oder nichtaromatischen, gegebenenfalls substituierten Kohlenwasserstoffrest darstellt, der gegebenenfalls durch ein oder mehrere Heteroatome und/oder durch eine oder mehrere Gruppen, die mindestens ein Heteroatom oder mindestens eine Gruppe, die mindestens ein Heteroatom umfasst, umfassen, unterbrochen ist, wobei R keine Hydroxyl-, primäre Amin-, sekundäre Amin-, Phosphat- oder Thiolgruppe umfasst, und einen Cyclus oder mehrere Cyclen, die optional kondensiert sind, enthalten kann,
wobei jede der Hydroxylgruppen des Ribose- oder Desoxyribose-Musters, die nicht an einer Bindung an das Antioxidationsmolekül oder an eine Lipidisierungsgruppe beteiligt ist, durch eine schützende Gruppe einer Hydroxylfunktion substituiert ist, die an das Sauerstoffatom der Hydroxylgruppe gebunden ist und aus den linearen, verzweigten und/oder cyclischen, gesättigten und/oder ungesättigten, aromatischen oder nichtaromatischen, gegebenenfalls substituierten Kohlenwasserstoffresten ausgewählt ist, die gegebenenfalls durch ein oder mehrere Heteroatome und/oder durch eine oder mehrere Gruppen, die mindestens ein Heteroatom oder mindestens eine Gruppe, die mindestens ein Heteroatom umfasst, mit Ausnahme der Hydroxyl-, primären Amin-, sekundären Amin-, Phosphat- und Thiolgruppen, umfassen, unterbrochen sind und einen Cyclus oder mehrere Cyclen, gegebenenfalls einen oder mehrere Heterocyclen, die ein oder mehrere Heteroatome umfassen, enthalten können, wobei die Cyclen optional kondensiert sind,
und wobei jede der primären Amingruppen, die nicht an einer Bindung an das Antioxidationsmolekül oder an eine Lipidisierungsgruppe beteiligt sind, durch eine schützende Gruppe einer primären Aminfunktion substituiert ist, die an das Stickstoffatom der primären Amingruppe gebunden ist und aus linearen, verzweigten und/oder cyclischen, gesättigten und/oder ungesättigten, aromatischen oder nichtaromatischen, gegebenenfalls substituierten Kohlenwasserstoffresten ausgewählt ist, die gegebenenfalls durch ein oder mehrere Heteroatome und/oder durch eine oder mehrere Gruppen, die mindestens ein Heteroatom oder mindestens eine Gruppe, die mindestens ein Heteroatom umfasst, mit Ausnahme der Hydroxyl-, primären Amin-, sekundären Amin-, Phosphat- und Thiolgruppen, umfassen, unterbrochen sind und einen Cyclus oder mehrere Cyclen, gegebenenfalls einen oder mehrere Heterocyclen, die ein oder mehrere Heteroatome umfassen, enthalten können, wobei die Cyclen optional kondensiert sind,
- oder eines der pharmazeutisch akzeptablen Salze der aktiven Verbindung.

2. Prodrug nach Anspruch 1, wobei die Lipidisierungsgruppe ausgewählt ist aus:
- den Gruppen -CH₂-R', -CO-CH₂-R' und -CO-(CH₂)₂-CO-O-R', wobei R' für Cholesterin, Tocopherol, Tocotrienol, einen Phenylkern, der gegebenenfalls durch eine oder mehrere R₅-Gruppen substituiert ist, einen Naphthylring, der gegenenfalls durch eine oder mehrere R₅-Gruppen substituiert ist, oder einen Pyridinring darstellt, der gegebenenfalls durch eine oder mehrere R₅-Gruppen substituiert ist, wobei R₅ eine lineare oder verzweigte, gesättigte oder ungesättigte C1-C25-Kohlenwasserstoffkette darstellt,
- einem Allylrest oder einem Propargylrest,
- den Gruppen -R und -CO-R, wobei R darstellt: eine lineare oder verzweigte, gesättigte oder ungesättigte C1-C25-Kohlenwasserstoffkette; eine Gruppe -(CH₂)₂-O-R₅-O-CH₃, wobei R₅ eine lineare oder verzweigte, gesättigte oder ungesättigte C1-C25-Kohlenwasserstoffkette darstellt; eine Gruppe -CH₂-CH(O-R₆)-O-R₇, wobei R₆ und R₇, gleich oder unterschiedlich, jeweils eine lineare oder verzweigte, gesättigte oder ungesättigte C1-C25-Kohlenwasserstoffkette darstellen; eine Gruppe -CH(O-R₆)-O-R₇, wobei R₆ und R₇, gleich oder unterschiedlich, jeweils eine lineare oder verzweigte, gesättigte oder ungesättigte C1-C25-Kohlenwasserstoffkette darstellen; oder eine Gruppe -CH₂-CH(O-CO-R₆)-O-CO-R₇, wobei R₆ und R₇, gleich oder unterschiedlich, jeweils eine lineare oder verzweigte, gesättigte oder ungesättigte C1-C25-Kohlenwasserstoffkette darstellen,
- den Gruppen -CH₂-R₈, wobei R₈ eine Triazolgruppe, eine Benzothiophengruppe, eine Benzofurangruppe oder eine Indolgruppe darstellt, wobei R₈ gegebenenfalls durch eine oder mehrere R₅-Gruppen ersetzt wird, wobei R₅ eine lineare oder verzweigte, gesättigte oder ungesättigte C1-C25-Kohlenwasserstoffkette darstellt,
- den Gruppen -(CH₂)₂-O-(CH₂)₂-O-(CH₂)ₚ-O-CH₂-R₁₅, wobei p eine ganze Zahl zwischen 2 und 25 ist und R₁₅ ein Wasserstoffatom oder eine Phenylgruppe darstellt;
- den Gruppen -CO-CH₂-O-(CH₂)_{q}-O-CH₂-R₁₆, wobei q eine ganze Zahl zwischen 2 und 25 ist und R₁₆ ein Wasserstoffatom oder eine Phenylgruppe darstellt;
- einer Triazolgruppe, die gegebenenfalls durch eine lineare oder verzweigte, gesättigte oder ungesättigte C1-C25-Kohlenwasserstoffkette substituiert ist.

3. Prodrug nach einem der Ansprüche 1 oder 2, wobei die Kopplung des Antioxidationsmoleküls an das Nukleosid an einem ersten Sauerstoffatom des Nukleosids durchgeführt wird, das aus dem Sauerstoffatom an der Position 5' des Ribose- oder Desoxyribose-Musters und dem Sauerstoffatom an der Position 3' des Ribose- oder Desoxyribose-Musters ausgewählt ist.

4. Prodrug nach Anspruch 3, wobei die Lipidisierungsgruppe von einem zweiten Sauerstoffatom des Nukleosids, das sich von dem ersten Sauerstoffatom unterscheidet, ausgewählt aus dem Sauerstoffatom, das sich in der Position 5' des Ribose- oder Desoxyribose-Musters befindet, und dem Sauerstoffatom, das sich in der Position 3' des Ribose- oder Desoxyribose-Musters befindet, und/oder von einem Stickstoffatom einer Amingruppe der Nukleobase getragen wird.

5. Prodrug nach einem der Ansprüche 1 bis 4, wobei das Nukleosid die allgemeine Formel (I) aufweist: 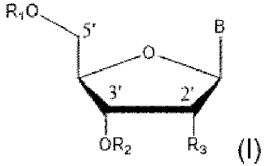 wobei
B die Nukleobase darstellt, die gegebenenfalls an einer Amingruppe durch eine Lipidisierungsgruppe substituiert ist, wobei jede der möglichen primären Amingruppen der Nukleobase, die nicht an einer Bindung an das Antioxidationsmolekül oder an eine Lipidisierungsgruppe beteiligt ist, durch eine schützende Gruppe einer primären Aminfunktion gemäß Anspruch 1 substituiert ist,
und
- entweder R₁ die Bindung an das Antioxidationsmolekül darstellt, und
R₂ eine Lipidisierungsgruppe darstellt,
R₃ H oder OR₄ darstellt, wobei R₄ eine Lipidisierungsgruppe oder eine schützende Gruppe einer Hydroxylfunktion gemäß Anspruch 1 darstellt,
oder R₂ und R₄ zusammen mit den Sauerstoffatomen, an die sie gebunden sind, eine Acetalgruppe bilden, die eine oder mehrere lineare, verzweigte und/oder cyclische, gesättigte oder ungesättigte, aromatische oder nichtaromatische, gegebenenfalls substituierte Kohlenwasserstoffketten umfasst, die gegebenenfalls durch ein oder mehrere Heteroatome und/oder durch eine oder mehrere Gruppen, die mindestens ein Heteroatom oder mindestens eine Gruppe, die mindestens ein Heteroatom umfasst, mit Ausnahme der Hydroxyl-, primären Amin-, sekundären Amin-, Phosphat- und Thiolgruppen, umfassen, unterbrochen sind und einen Cyclus oder mehrere Cyclen, gegebenenfalls einen oder mehrere Heterocyclen, die ein oder mehrere Heteroatome umfassen, enthalten können, wobei die Cyclen optional kondensiert sind,
- oder R₁ die Bindung an das Antioxidationsmolekül darstellt, und
R₂ eine Lipidisierungsgruppe oder eine schützende Gruppe einer Hydroxylfunktion gemäß Anspruch 1 darstellt,
R₃ OR₄ darstellt, wobei R₄ eine Lipidisierungsgruppe darstellt,
- entweder R₂ die Bindung an das Antioxidationsmolekül darstellt, und
R₁ eine Lipidisierungsgruppe darstellt,
R₃ H oder OR₄ darstellt, wobei R₄ eine Lipidisierungsgruppe oder eine schützende Gruppe einer Hydroxylfunktion gemäß Anspruch 1 darstellt,
- oder R₂ die Bindung an das Antioxidationsmolekül darstellt, und
R₁ eine Lipidisierungsgruppe oder eine schützende Gruppe einer Hydroxylfunktion gemäß Anspruch 1 darstellt,
R₃ OR₄ darstellt, wobei R₄ eine Lipidisierungsgruppe darstellt.

6. Prodrug nach einem der Ansprüche 1 bis 5, wobei das Antioxidationsmolekül über einen Spacerarm, der mindestens eine Carbonsäureestergruppe und/oder mindestens eine Carbonylgruppe enthält, die an das Sauerstoffatom in Position 5' des Ribose- oder Desoxyribose-Musters oder an das Sauerstoffatom in Position 3' des Ribose- oder Desoxyribose-Musters oder an ein Sauerstoffatom des Antioxidationsmoleküls gebunden ist, an das Nukleosid gekoppelt ist.

7. Prodrug nach einem der Ansprüche 1 bis 6, wobei das Antioxidationsmolekül über einen Spacerarm der allgemeinen Formel (II) kovalent an das Nukleosid gekoppelt ist: 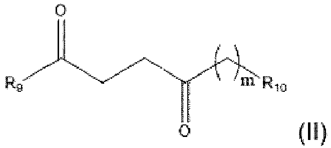 wobei R₉ das Antioxidationsmolekül darstellt und R₁₀ das Nukleosid darstellt, und
- m gleich 0 ist und die Bindung des Antioxidationsmoleküls an den Spacerarm von einem Sauerstoffatom des Antioxidationsmoleküls getragen wird und/oder die Bindung des Nukleosids an den Spacerarm von einem Sauerstoffatom des Nukleosids getragen wird,
- oder m gleich 1 ist und die Bindung des Antioxidationsmoleküls an den Spacerarm von einem Sauerstoffatom des Antioxidationsmoleküls getragen wird.

8. Prodrug nach einem der Ansprüche 1 bis 7, wobei das Antioxidationsmolekül aus α-Tocopherol, β-Tocopherol, γ-Tocopherol, α-Tocotrienol, β-Tocotrienol, γ-Tocotrienol, δ-Tocotrienol, Resveratrol, Liponsäure, Retinol, Retinal, Retinsäure und Ascorbinsäure ausgewählt ist.

9. Pharmazeutische Zusammensetzung, **dadurch gekennzeichnet, dass** sie ein Prodrug nach einem der Ansprüche 1 bis 8 in einem pharmazeutisch akzeptablen Trägermittel enthält.

10. Pharmazeutische Zusammensetzung nach Anspruch 9, in Form einer Emulsion, die eine lipophile Phase und eine wässrige Phase umfasst, wobei das Prodrug in der lipophilen Phase enthalten ist, vorzugsweise in Form einer Nanoemulsion.

11. Prodrug nach einem der Ansprüche 1 bis 8 oder pharmazeutische Zusammensetzung nach einem der Ansprüche 9 bis 10 zur Verwendung als Arzneimittel.

12. Prodrug oder pharmazeutische Zusammensetzung zur Verwendung nach Anspruch 11 zur Behandlung chemischer oder Strahlenverbrennungen.

13. Prodrug oder pharmazeutische Zusammensetzung zur Verwendung nach Anspruch 12, wobei das Prodrug oder die pharmazeutische Zusammensetzung topisch verabreicht wird.

14. Prodrug oder pharmazeutische Zusammensetzung zur Verwendung nach Anspruch 11 zur Behandlung einer neurodegenerativen Erkrankung.

15. Prodrug oder pharmazeutische Zusammensetzung zur Verwendung nach Anspruch 14, wobei das Prodrug oder die pharmazeutische Zusammensetzung oral oder parenteral verabreicht wird.

## Claims

1. Prodrug, **characterised in that** it consists of:
- a so-called active compound comprising:
a pharmaceutically-active antioxidant molecule covalently coupled, by a bond comprising at least one carboxylic ester group, to a nucleoside comprising a ribose or deoxyribose unit covalently bonded to a nucleic base,
said nucleoside bearing at least one group, referred to as a lipidising group, selected from among the groups -R and-CO-R, wherein R represents a linear, branched and/or cyclic, saturated and/or unsaturated, aromatic or non-aromatic, hydrocarbon radical, optionally substituted, optionally interrupted by one or more heteroatom(s) and/or by one or more group(s) comprising at least one heteroatom or at least one group comprising at least one heteroatom, R comprising no hydroxyl, primary amine, secondary amine, phosphate or thiol group, and possibly including one cycle or several cycles, optionally condensed, each of the hydroxyl groups of said ribose or deoxyribose unit not involved in a bond to said antioxidant molecule or to a lipidising group being substituted by a protective group of a hydroxyl function attached to the oxygen atom of said hydroxyl group and selected from among linear, branched and/or cyclic, saturated and/or unsaturated, aromatic or non-aromatic, hydrocarbon radicals, optionally substituted, optionally interrupted by one or more heteroatom(s) and/or by one or more group(s) comprising at least one heteroatom or at least one group comprising at least one heteroatom, excluding hydroxyl, primary amine, secondary amine, phosphate and thiol groups, and possibly including one cycle or several cycles, optionally one or more heterocycle(s) comprising one or more heteroatom(s), said cycles being optionally condensed,
and each of the primary amine groups not involved in a bond to said antioxidant molecule or to a lipidising group being substituted by a protective group of a primary amine function attached to the nitrogen atom of said primary amine group and selected from among linear, branched and/or cyclic, saturated and/or unsaturated, aromatic or non-aromatic, hydrocarbon radicals, optionally substituted, optionally interrupted by one or more heteroatom(s) and/or by one or more group(s) comprising at least one heteroatom or at least one group comprising at least one heteroatom, excluding hydroxyl, primary amine, secondary amine, phosphate and thiol groups, and which may include one cycle or several cycles, optionally one or more heterocycle(s) comprising one or more heteroatom(s), said cycles being optionally condensed,
- or one of the pharmaceutically-acceptable salts of said active compound.

2. Prodrug according to claim 1, wherein said lipidising group is selected from among:
- the groups -CH₂-R', -CO-CH₂-R' and -CO-(CH₂)₂-CO-O-R', where R' represents cholesterol, tocopherol, tocotrienol, a phenyl ring possibly substituted by one or more group(s) R₅, a naphthyl ring possibly substituted by one or more group(s) R₅, or a pyridine ring possibly substituted by one or more group(s) R₅, wherein R₅ represents a linear or branched, saturated or unsaturated, C1-C25 hydrocarbon chain,
- an allyl radical or a propargyl radical,
- the groups R and-CO-R, where R represents: a linear or branched, saturated or unsaturated, C1-C25 hydrocarbon chain; a group -(CH₂)₂-O-R₅-O-CH₃ where R₅ represents a linear or branched, saturated or unsaturated, C1-C25 hydrocarbon chain; a group -CH₂-CH(O-R₆)-O-R₇ where R₆ and R₇, identical or different, each represent a linear or branched, saturated or unsaturated, C1-C25 hydrocarbon chain; a group -CH(O-R₆)-O-R₇ where R₆ and R₇, identical or different, each represent a linear or branched, saturated or unsaturated, C1-C25 hydrocarbon chain; or a group -CH₂-CH(O-CO-R₆)-O-CO-R₇ where R₆ and R₇, identical or different, each represent a linear or branched, saturated or unsaturated, C1-C25 hydrocarbon chain,
- the groups -CH₂-R₈ where R₈ represents a triazole group, a benzothiophene group, a benzofuran group or an indole group, R₈ being optionally substituted by one or more group(s) R₅ where R₅ represents a linear or branched, saturated or unsaturated, C1-C25 hydrocarbon chain,
- the groups -(CH₂)₂-O-(CH₂)₂-O-(CH₂)ₚ-O-CH₂-R₁₅, where p is an integer comprised between 2 and 25 and R₁₅ represents a hydrogen atom or a phenyl group;
- the groups -CO-CH₂-O-(CH₂)_{q}-O-CH₂-R₁₆, where q is an integer comprised between 2 and 25 and R₁₆ represents a hydrogen atom or a phenyl group;
- a triazole group, optionally substituted by a linear or branched, saturated or unsaturated, C1-C25 hydrocarbon chain.

3. Prodrug according to any one of claims 1 or 2, wherein the coupling of said antioxidant molecule to said nucleoside is carried out on a first oxygen atom of said nucleoside selected from among the oxygen atom located in the position 5' of said ribose or deoxyribose unit and the oxygen atom located in the position 3' of said ribose or deoxyribose unit.

4. Prodrug according to claim 3, wherein said lipidising group is carried by a second oxygen atom of said nucleoside, different from said first oxygen atom, selected from among the oxygen atom located in the position 5' of said ribose or deoxyribose unit and the oxygen atom located in the position 3' of said ribose or deoxyribose unit, and/or by a nitrogen atom of an amine group of said nucleic base.

5. Prodrug according to any one of claims 1 to 4, wherein said nucleoside has the general formula (I): 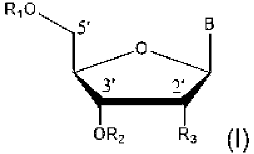 wherein
B represents said nucleic base, possibly substituted at an amine group by a lipidising group, each of the possible primary amine groups of said nucleic base not involved in a bond to said antioxidant molecule or to a lipidising group being substituted by a protective group of a primary amine function as defined in claim 1,
and
- either R₁ represents the bond to said antioxidant molecule, and
R₂ represents a lipidising group,
R₃ represents H or OR₄ where R₄ represents a lipidising group or a protective group of a hydroxyl function as defined in claim 1,
or R₂ and R₄ form together, with the oxygen atoms to which they are attached, an acetal group comprising one or more linear, branched and/or cyclic, saturated or unsaturated, aromatic or non-aromatic, hydrocarbon chain(s), optionally substituted, optionally interrupted by one or more heteroatom(s) and/or by one or more group(s) comprising at least one heteroatom or at least one group comprising at least one heteroatom, excluding hydroxyl, primary amine, secondary amine, phosphate and thiol groups, and possibly including one or more cycle(s), optionally one or more heterocycle(s) comprising one or more heteroatom(s), the cycles being optionally condensed,
- or R₁ represents the bond to said antioxidant molecule, and
R₂ represents a lipidising group or a protective group of a hydroxyl function as defined in claim 1,
R₃ represents OR₄ where R₄ represents a lipidising group,
- or R₂ represents the bond to said antioxidant molecule, and
R₁ represents a lipidising group,
R₃ represents H or OR₄ where R₄ represents a lipidising group or a protective group of a hydroxyl function as defined in claim 1,
- or R₂ represents the bond to said antioxidant molecule, and
R₁ represents a lipidising group or a protective group of a hydroxyl function as defined in claim 1,
R₃ represents OR₄ where R₄ represents a lipidising group.

6. Prodrug according to any one of claims 1 to 5, wherein said antioxidant molecule is covalently coupled to said nucleoside via a spacer arm containing at least one carboxylic ester group and/or at least one carbonyl group bonded to the oxygen atom at the position 5' of said ribose or deoxyribose unit or to the oxygen atom at the position 3' of said ribose or deoxyribose unit or to an oxygen atom of said antioxidant molecule.

7. Prodrug according to any one of claims 1 to 6, wherein said antioxidant molecule is covalently coupled to said nucleoside via a spacer arm of general formula (II): 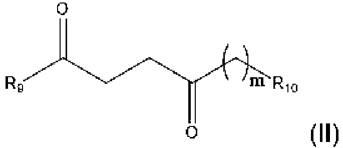 wherein R₉ represents the antioxidant molecule and R₁₀ represents the nucleoside, and
- m is equal to 0 and the bond of the antioxidant molecule to the spacer arm is carried by an oxygen atom of the antioxidant molecule and/or the bond of the nucleoside to the spacer arm is carried by an oxygen atom of the nucleoside,
- or m is equal to 1 and bonding of the antioxidant molecule to the spacer arm is carried by an oxygen atom of the antioxidant molecule.

8. Prodrug according to any one of claims 1 to 7, wherein said antioxidant molecule is selected from among α-tocopherol, β-tocopherol, γ-tocopherol, α-tocotrienol, β-tocotrienol, γ-tocotrienol, δ-tocotrienol, resveratrol, lipoic acid, retinol, retinal, retinoic acid and ascorbic acid.

9. Pharmaceutical composition **characterised in that** it contains a prodrug according to any one of claims 1 to 8 in a pharmaceutically-acceptable vehicle.

10. Pharmaceutical composition according to claim 9, in the form of an emulsion comprising a lipophilic phase and an aqueous phase, said prodrug being contained in said lipophilic phase, preferably in the form of a nanoemulsion.

11. Prodrug according to any one of claims 1 to 8 or pharmaceutical composition according to any one of claims 9 to 10 for its use as a medicine.

12. Prodrug or pharmaceutical composition for its use according to claim 11, for the treatment of chemical or radiological burns.

13. Prodrug or pharmaceutical composition for its use according to claim 12, wherein said prodrug or pharmaceutical composition is administered topically.

14. Prodrug or pharmaceutical composition for its use according to claim 11, for the treatment of a neurodegenerative disease.

15. Prodrug or pharmaceutical composition for its use according to claim 14, wherein said prodrug or pharmaceutical composition is administered orally or parenterally.
